(19) Europäisches Patentamt
European Patent Office
Office européen des brevets

(11) **EP 3 024 470 B1**

(12) **EUROPÄISCHE PATENTSCHRIFT**

(45) Veröffentlichungstag und Bekanntmachung des Hinweises auf die Patenterteilung:
**01.01.2020  Patentblatt 2020/01**

(21) Anmeldenummer: **14758068.2**

(22) Anmeldetag: **23.07.2014**

(51) Int Cl.:
**A61K 36/40** *(2006.01)*      **A61P 1/00** *(2006.01)*
**A61P 1/12** *(2006.01)*

(86) Internationale Anmeldenummer:
**PCT/AT2014/000148**

(87) Internationale Veröffentlichungsnummer:
**WO 2015/010144 (29.01.2015 Gazette 2015/04)**

(54) **ZUSATZSTOFF FÜR FUTTERMITTEL, NAHRUNGSMITTEL, TRINKWASSER ODER PHARMAZEUTISCHE PRÄPARATE**

ADDITIVE FOR ANIMAL FEED, FOODSTUFFS, DRINKING WATER OR PHARMACEUTICAL PREPARATIONS

ADJUVANT POUR ALIMENTS POUR ANIMAUX, DENRÉES ALIMENTAIRES, EAU POTABLE OU PRÉPARATIONS PHARMACEUTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.07.2013  AT 2382013 U**

(43) Veröffentlichungstag der Anmeldung:
**01.06.2016  Patentblatt 2016/22**

(73) Patentinhaber: **Erber Aktiengesellschaft
3131 Getzersdorf bei Traismauer (AT)**

(72) Erfinder:
• **TEICHMANN, Klaus**
  **A-3107 Viehofen (AT)**
• **HESSENBERGER, Sabine**
  **A-3430 Tulln (AT)**
• **SCHAUERHUBER, Cornelia**
  **A-3462 Absdorf (AT)**
• **PFEFFER, Martin**
  **A-3430 Tulln (AT)**
• **BINDER, Eva, Maria**
  **A-3430 Tulln (AT)**
• **SCHATZMAYR, Gerd**
  **A-3430 Tulln (AT)**

(74) Vertreter: **Cunow, Gerda
Cunow Patentanwalts KG
Teschnergasse 33/1/3
1180 Wien (AT)**

(56) Entgegenhaltungen:
• **DATABASE TCM [Online] SIPO; 4. Januar 2012 (2012-01-04), Li Yan et al.: "A kind of core of Fructus Corni extract, and its application in the preparation of composition with blood pressure lowering effect", XP002730799, Database accession no. CN-201210000983-A & CN 102 688 275 A (UNIV HENAN SCIENCE & TECH) 26. September 2012 (2012-09-26)**
• **DATABASE WPI Week 201312 Thomson Scientific, London, GB; AN 2012-M24241 XP002730800, & CN 102 558 281 A (UNIV HENAN SCI & TECHNOLOGY) 11. Juli 2012 (2012-07-11)**
• **DATABASE WPI Week 200914 Thomson Scientific, London, GB; AN 2009-F06163 XP002730801, & KR 2008 0098128 A (GIMPO CHUCKHYUP) 7. November 2008 (2008-11-07)**
• **MENKOVIC N ET AL: "Ethnobotanical study on traditional uses of wild medicinal plants in Prokletije Mountains (Montenegro)", JOURNAL OF ETHNOPHARMACOLOGY, Bd. 133, Nr. 1, 7. Januar 2011 (2011-01-07), Seiten 97-107, XP027575276, ELSEVIER IRELAND LTD, IE ISSN: 0378-8741 [gefunden am 2010-12-24]**
• **LEE JUN ET AL: "Galloyl Glucoses from the Seeds of Cornus officinalis with Inhibitory Activity against Protein Glycation, Aldose Reductase, and Cataractogenesis ex Vivo", BIOLOGICAL & PHARMACEUTICAL BULLETIN, Bd. 34, Nr. 3, März 2011 (2011-03), Seiten 443-446, XP009180566, ISSN: 0918-6158**

- **VAREED S K ET AL: "Anthocyanins in Cornus alternifolia, Cornus controversa, Cornus kousa and Cornus florida fruits with health benefits", LIFE SCIENCES, Bd. 78, Nr. 7, 11. Januar 2006 (2006-01-11), Seiten 777-784, XP025191083, PERGAMON PRESS, OXFORD, GB ISSN: 0024-3205, DOI: 10.1016/J.LFS.2005.05.094 [gefunden am 2006-01-11]**
- **DATABASE WPI Week 201023 Thomson Scientific, London, GB; AN 2010-A68142 XP002730803, & KR 2010 0000728 A (AMOREPACIFIC CORP) 6. Januar 2010 (2010-01-06)**
- **DATABASE WPI Week 200654 Thomson Scientific, London, GB; AN 2006-522847 XP002730804, & CN 1 730 057 A (QIU G) 8. Februar 2006 (2006-02-08)**

Bemerkungen:
Die Akte enthält technische Angaben, die nach dem Eingang der Anmeldung eingereicht wurden und die nicht in dieser Patentschrift enthalten sind.

## Beschreibung

Die vorliegende Erfindung bezieht sich auf einen Zusatzstoff für Futtermitteln, Nahrungsmitteln, Trinkwasser oder pharmazeutischen Präparaten, hergestellt aus wenigstens einer Kornelkirschen-Spezies, insbesondere *Cornus mas, Cornus officinalis, Cornus chinensis* oder *Cornus eydeana.*

Inhaltsstoffe und Wirkung der Kerne von Kornelkirschen-Früchten sind in der Literatur kaum beschrieben. Lee et al., Biological & pharmaceutical bulletin, 2011, 34(3), 443-6 beschreiben eine mögliche inhibierende Wirkung von Inhaltsstoffen *ex vivo* von C. *officinalis* Kernen auf das Enzym Aldose Reduktase, auf die Proteinglyakierung und auf die Entstehung von grauem Star im Zusammenhang mit Diabetes. Abseits davon ist kein Zusammenhang von Kornelkirschen-Kernen mit anderen Erkrankungen, insbesondere nicht mit Erkrankungen des Magen-Darm Traktes, bekannt. Wirtschaftlich betrachtet gibt es bis dato keine relevante Verwertungsmöglichkeit für Kornelkirschen-Kerne.

Aus Li Yan et al.: «A kind of core of Fructus Corni extract, and its application in the preparation of composition with blood presure lowering effect» Database accession Nr. AN-CN-201210000983-A sind Extrakte von Kornelkirschen-Kernen zur Herstellung von Blutdruck senkenden Präparaten bekannt geworden.

Aus KR 2008098128 A ist die Verwendung von Kornelkirschen in einer Mischung mit Getreide, Piniennadeln und dgl. bekannt geworden, welche Mischung mit Hefe fermentiert und getrocknet wird und als Futtermittelzusatz verwendet wird.

N. Menkovic et al., Journal of Ethnopharmacology 133 (2011) 97-107, sind eine Vielzahl von wilden Medizinpflanzen, welche in dem Bergen Montenegros wachsen, entnehmbar, sowie der mögliche Einsatz dieser Pflanzen. Unter diesen Pflanzen ist unter anderem *Cornus mas* erwähnt.

Die DE19938931A1 beschreibt die Verwendung von insbesondere einem metabolischen Extrakt der Früchte von *Cornus officinalis* als Insektizid zur Vertilgung oder Bekämpfung von insbesondere Nutzholz schädigenden Insekten, wie Termiten.

Das Fruchtfleisch diverser Cornus Arten findet teilweise in der Lebensmittel- oder Gesundheitsindustrie und in privaten Haushalten Anwendung. In der asiatischen Medizin wird das Fruchtfleisch beispielsweise zur Behandlung von gastrointestinalen Beschwerden und Fieber eingesetzt. Fruchtfleisch von C. *officinalis* wird in der Traditionellen Chinesischen Medizin verwendet und ist für seine tonisierende, analgetische und diuretische Aktivität bekannt. Vareed et al., Life sciences, 2006, 78(7), 777-84 beschreiben antibakterielle, anti-Histamin, anti-allergische und anti-Malaria Aktivitäten des Fruchtfleisches.

Unter den Bezeichnungen "Kornelkirschen", "Kornelkirschen Spezies" oder "Kornelkirschen-Gruppe" werden die nahe verwandten *Cornus* Spezies *Cornus officinalis, Cornus mas, Cornus chinensis, Cornus eydenea, Cornus volkensii* und *Cornus sessilis* zusammengefasst. Die nahe Verwandtschaft innerhalb der Kornelkirschen-Gruppe ist in der Literatur ausführlich beschrieben (siehe z.B. Qiu-Yun (Jenny) Xiang, Evolution, 2005, 59(8), 1685-1700). Als Merkmal zur Bestimmung des Verwandtschaftsgrades können unter anderem morphologische Eigenschaften wie zum Beispiel Form und Anordnung des Deckblatts, Infloreszenztyp, Entwicklungsstatus der winterlichen Blütenstandsknospen, Fruchtart oder Fruchtfarbe, aber im Besonderen auch Chromosomenanzahl und phylogenetische Eigenschaften, wie zum Beispiel die Sequenzhomologie der 26S rDNA herangezogen werden.

In der Literatur und in der Umgangssprache, speziell im ländlichen Raum, sind eine Vielzahl von Synonymen für einzelne Vertreter der Kornelkirschen-Gruppe bekannt, wobei beispielhaft einige gängige Bezeichnungen und/oder Synonyme für die jeweilige *Cornus* Spezies aufgelistet werden. Für *"Cornus mas"* sind dies z.B C. *mas* (L).; *Macrocarpium* mas; Gelber Hartriegel; Europäischer Hartriegel; Europäische Kornelkirsche; Kornelen; Welsche Kirsche; für *"Cornus officinalis"* sind dies z.B C. *officinalis* (SIEB et, Zucc); *Cornus mascula;* asiatischer Hartriegel, chinesischer Hartriegel, asiatische Kornelkirsche; chinesische Kornelkirsche; für *"Cornus chinensis"* sind dies z.B. C. *chinensis* (WANGERIN), *Macrocarpium* chinense, chinesische Kornelkirsche, chinesischer Hartriegel; für Cornus eydeana sind dies z.B. C. eydeana (XIANG), Eyde's Hartriegel oder Eyde's Kornelkirsche.

Als Wirksubstanzen werden aus Kornelkirschen-Kernen isolierte Substanzen verstanden, die also solche eine antibakterielle und/oder antivirale Wirkung oder in Kombination mit Kornelkirschen-Kernmehl oder Kornelkirschen-Kernextrakten eine synergistische antibakterielle Wirkung zeigen. Als isolierte Wirksubstanzen werden solche verstanden, die in einer Reinheit von wenigstens 70 Gew.-%, bevorzugt ≥ 80 Gew.-%, insbesondere ≥ 90 Gew.-% vorliegen.

In der Nutztierzüchtung sind gesunde Tiere die Grundlage einer hohen Produktivität des landwirtschaftlichen Betriebs. Seit dem EU-weiten Verbot von antibiotischen Leistungsförderern in Futtermitteln im Jahr 2006 ist man auf alternative Präparate zur Gesundheitsförderung und Leistungssteigerung angewiesen.

Kritische Phasen der Tieraufzucht, wie beispielsweise bei der Nahrungsumstellung oder der Umstallung, verursachen den Tieren Stress, der sich nachteilig auf das Immunsystem auswirkt. Das Immunsystem eines jungen Tieres ist noch nicht ausgereift, was ubiquitäre, opportunistische Krankheitserreger zusätzlich begünstigt. Diese können sich an die Darmwand anheften und diese zerstören, was zu einer negativen Beeinflussung der Darmarchitektur, wie einer verringerten resorptiven Oberfläche, führt. Das kann in weiterer Folge zu allergischen Reaktionen, Septikämie und den Anzeichen eines endotoxischen Schocks führen. Magen-Darm-Erkrankungen, im Besonderen Durchfallerkrankungen, sind in diesem Zusammenhang ein großes Problem, da sie die Entwicklung, Gesundheit und Leistung der Tiere stark negativ beeinflussen.

Speziell bei Jungtieren ist das Verdauungssystem noch nicht ausreichend entwickelt, sodass die Verdauung fester Nahrung unvollständig abläuft. Der Magen produziert noch nicht ausreichend Magensäure, was grundsätzlich säureempfindlichen Pathogenen, wie z.B. Salmonellen nach Aufnahme die Magenpassage ermöglicht und zu einer unzureichenden Spaltung von höhermolekularen, polymeren Nahrungsbestandteilen führt. Zusammen mit geringer Bildung von Verdauungssäften und -enzymen führt dies dazu, dass relativ große Mengen an unvollständig verdauten Nahrungsbestandteilen im Darm für Mikroorganismen zur Verfügung stehen.

Bei Mastschweinen gelten Ileitis und Colitis als die relevantesten Durchfallursachen. Als bakterielle Erreger sind Spirochäten, wie beispielsweise *Brachyspira psilosicoli,* pathogene *Escherichia coli* Stämme, Kokzidien, Clostridien und Salmonellen bekannt. Ein Befall von *Lawsonia intracellularis* kann zu Verdickungen, Nekrosen, Blutungen und lokaler Entzündung des Dünndarms führen.

Mehr als 2000 Serotypen von Salmonella-Spezies können zwischen Tieren und Menschen übertragen werden, wobei S. *typhimurium* und S. *enteritidis* zu den häufigsten vom Tier auf den Menschen übertragenen Erregern von Durchfallerkrankungen gehören. Die Salmonellenkontrolle bei Nutztieren ist wichtig, um infektiöse Darmerkrankungen bei den Nutztieren zu verhindern und um die Krankheitsübertragung auf den Menschen zu verhindern. Letzteres gilt auch für Campylobacterarten, wie beispielsweise *C. jejuni,* die in der Regel nicht pathogen für die Nutztiere selbst sind, aber für schwere Durchfallerkrankungen beim Menschen verantwortlich sind. Viele Salmonellenarten können Durchfall, Enteritis und Septikämie, vor allem bei Jungtieren, verursachen, oder ohne klinische Symptome vorkommen, wobei sie Wachstum und Futterverwertung der Nutztiere beeinträchtigen können. Colibazillose oder Coliseptikämie ist weltweit die häufigste Infektionskrankheit bei Geflügel. Sie wird von pathogenen Stämmen des Bakteriums *E. coli* verursacht. *coli* Stämmen und den von ihnen produzierten Toxinen. Die durch Durchfall verursachte geringere Wasser- und Nährstoffaufnahme stellt ein großes Problem dar, da Dehydrierung rasch zu Gewichtsverlust und zum Tod der Jungtiere führen kann.

Weitere Beispiele für bakterielle Krankheitserreger sind Clostridien, welche mit Krankheiten wie nekrotischer Enteritis und Dermatitis assoziiert werden. Streptokokken beim Schwein haben Bedeutung als Erreger von Meningitis, Septikämie, Arthritis und Krankheiten der Atemwege.

Neben bakteriellen Erregern spielen auch Protozoen eine Rolle als Durchfallerreger. Die Kokzidiose bei Geflügel, verursacht durch Vertreter der Gattung *Eimeria,* insbesondere *E. tenella,* ist eine der wichtigsten Geflügelkrankheiten. Kokzidien verursachen unter anderem Durchfall, eine schlechte Nahrungsverwertung, eine verzögerte Tierentwicklung und können, je nach Art und Stamm des Erregers, auch zum Tod führen. Häufig kommen Sekundärinfektionen durch bakterielle Erreger, wie Clostridien oder pathogene *E. coli* Stämmen vor. Aber auch bei anderen Nutztieren wie Rindern (*Eimeria zuernii, Eimeria bovis*)*,* Schafen und Saugferkeln (*Isospora* Arten) kommen Kokzidien vor. Die Bekämpfung der Kokzidienerreger beruht hauptsächlich auf der prophylaktischen Gabe von Kokzidiostatika. Diese verursachen das Auftreten von Resistenzen, sodass alternative Lösungen zur Bekämpfung der Kokzidiose daher von höchster Wichtigkeit sind.

Neben bakteriellen Erregern und Protozoen können auch zahlreiche Viren alleine oder im Zusammenwirken mit anderen Erregern Durchfallerkrankungen verursachen. Bei Ferkeln sind vor allem Coronaviren und Rotaviren als Auslöser für Durchfallerkrankungen von Relevanz. Da in der Praxis häufig mehrere durchfallregende Pathogene gleichzeitig auftreten, ist ein Mittel mit breiter Wirkung wünschenswert.

Trotz guter Hygienebedingungen und gutem Stallmanagement sind immer wieder auftretende Erkrankungen wie Enteritis, Ileitis, Colitis, Septikämie, Colibazillose, Coliseptikämie, Dermatitis, Meningitis, Septikämie, Arthritis, Kokzidiose oder Erkrankungen der Atemwege von Nutztieren nicht vermeidbar. Diese Erkrankungen stehen in einem direkten Zusammenhang mit einem Befall durch die oben beschriebenen pathogenen Organismen oder Viren. Erkrankte Tiere zeigen in der Regel eine verminderte Leistungsfähigkeit, die sich beispielsweise durch ein erhöhtes Durchfallgeschehen, eine Reduktion der Gewichtszunahme oder eine Verschlechterung der Futterverwertung auszeichnet.

Das grundlegende Problem ist, dass bestehende Produkte nicht ausreichend zur Prävention und/oder zur Behandlung der oben genannten Erkrankungen, im Besonderen Durchfallerkrankungen, geeignet sind, wobei vor allem der präventive Einsatz von Antibiotika, Kokzidiostatika sowie von Zink zur Durchfallbehandlung in der EU verboten ist.

Die vorliegende Erfindung zielt nun darauf ab, einen Zusatzstoff zur Verfügung zu stellen der natürlichen Ursprungs und ökologisch unbedenklich ist, und welcher zur Prävention und/oder Behandlung von mikrobiell oder viral verursachten Erkrankungen, bevorzugt Erkrankungen des Magen-Darm-Traktes, im Besonderen Durchfallerkrankungen, als auch zur Leistungssteigerung von Nutztieren sowie zur Desinfektion eingesetzt werden kann.

Zur Lösung dieser Aufgabe ist die vorliegende Erfindung dadurch gekennzeichnet, dass wenigstens eine Komponente gewählt aus Kornelkirschen-Kernmehl und/oder Kornelkirschen-Kernextrakt sowie zusätzlich wenigstens eine weitere aus chemischer Synthese und/oder mikrobieller Fermentation erhaltene Substanz, gewählt aus Kalziumformiat, Benzoesäure, Ascorbinsäure, Ameisensäure oder Vitamin B12 und gegebenenfalls wenigstens ein Bestandteil gewählt aus Trägermaterialien, Formulierungshilfsstoffen und/oder biologisch wirksamen Komponenten enthalten sind. Mit einem derartigen Zusatzstoff gelingt es überaschenderweise, pathogene Mikroorganismen, wie beispielsweise *Salmonella typhimurium, Samonella enteritidis,* pathogene *Escherichia coli* Stämme O128:H2 und O8:K87(F4), *Campylobacter coli, Clostridium perfringens* Toxin Typ A, *Clostridium perfringens* Toxin Typ C, *Streptococcus suis,* in ihrem Wachstum zu hemmen. Die antibakterielle Wirkung, ausgedrückt als minimale Hemmkonzentration (MHK), durch welche das Wachstum um mindestens 50 % gehemmt wird, ist gegenüber pathogenen Stämmen zirka um das Zehnfache stärker als gegenüber nicht pathogenen Stämmen, wie beispielswiese den probiotischen Bakterien *Enterococcus faecium* und *Lactobacillus salivarius.* Indem der Zusatzstoff zusätzlich mindestens eine weitere aus chemischer Synthese und/oder mikrobieller Fermentation erhaltene Substanz, nämlich Kalziumformiat, Benzoesäure, Ascorbinsäure, Ameisensäure und/oder Vitamin B12 ist, wird eine synergistische Wirkung des Zusatzstoffes, insbesondere in Bezug auf deren anti-mikrobielle Wirkung erzielt.

Überraschenderweise hat sich gezeigt, dass die Kornelkirschen-Kerne eine wesentlich höhere antibakterielle Wirkung besitzen als das Kornelkirschen-Fruchtfleisch. Dabei wurde gefunden, dass die chemische Zusammensetzung des Kornelkirschen-Fruchtfleischs von jener des Kornelkirschen-Kerns nahezu vollständig verschieden ist und es de facto nur sehr wenige extrahierbaren Substanzen gibt, die in Fruchtfleisch und Kern gemeinsam vorkommen, und nahezu keine, die in vergleichbaren Mengen vorkommen. Beispielhaft sind hierfür Hydroxymethylfurfural sowie Pentagalloylglucose zu nennen, die ausschließlich im Kernextrakt gefunden wurden, Ellagsäure, die im Fruchtfleisch, im Gegensatz zum Kern, lediglich in Spuren nachgewiesen wurde und Gallussäure als Substanz, die in Kern und Fruchtfleisch in vergleichbaren Mengen detektierbar war. Des Weiteren zeigte eine analoge vergleichende Analyse von Kernextrakten aus *C. mas* und *C. officinalis* gleichartige HPLC-DAD Chromatogramme, weshalb die chemische Zusammensetzung der Kerne dieser sehr nahen verwandten Pflanzenspezies als identisch anzunehmen ist.

Pro Kilogramm Kornelkirschen-Kernextrakt konnten zirka 5000 mg Pentagalloylglucose, sowie überraschenderweise auch zirka 700 mg Gallussäure, zwischen 5700 mg und 10400 mg Ellagsäure, sowie zirka 500 mg Loganin und zwischen 10 mg und 170 mg Dihydroquercentin nachgewiesen werden. Allerdings wurde im wässrigen Extrakt keine Pentagalloylglucose und geringere Mengen an Dihydroquercentin aufgrund der schlechteren Löslichkeit in Wasser nachgewiesen. Der mengenmäßig größte Teil des Kernextraktes besteht jedoch aus Proteinen, Kohlenhydraten sowie Fetten.

Von Gallussäure, Pentagalloylglucose, sowie Ellagsäure sind antibakterielle Wirkungen und Vorkommen in Mitteln zur Bekämpfung von Durchfall bekannt. (Chen et al. 2006; Xiao et al. 2013; Verhelst et al. 2010; Funatogawa et al. 2004).

Loganin wirkt antiapoptotisch (Li et al. 2010), besitzt eine Hemmwirkung gegen *E. coli* (Graikou et al. 2002). Ebenso wurde eine antibakterielle Wirkung von Dihydroquercetin gegen Streptococcus sobrinus beschrieben. Jedoch können die im Kornelkirschen-Kernextrakt gefundenen geringen Konzentrationen von Loganin und Dihydroquercetin die beobachtete gute antibakterielle Wirkung des Kernmehls bzw. des Kernextrakts nicht erklären.

Die Reinsubstanzen Gallussäure, Ellagsäure, Pentagalloylglucose und Loganin zeigten überraschenderweise eine wesentlich schlechtere bakterielle Hemmwirkung von jeweils über 500 mg pro Liter im Vergleich zum Kernextrakt, dessen minimale Hemmkonzentration zwischen 78 und 156 mg pro Liter liegt.

Überraschenderweise zeigen diese Ergebnisse, dass die Pentagalloylglucose nicht wesentlich zur antibakteriellen Wirkung der Kornelkirschenkern-Produkte beiträgt, da die ethanolischen und die wässrigen Extrakte annähernd die gleichen minimalen Hemmkonzentrationen für E. coli O128:H2, S. typhimurium und C. perfringens Toxin Typ A (siehe Beispiel 4) aufweisen, die Substanz Pentagalloylglucose jedoch aufgrund der Unlöslichkeit in Wasser nur in den ethanolischen Extrakten vorkommt.

Bei Kernen anderer Früchte, deren Fruchtfleisch eine antibakterielle Wirkung besitzen, wie z.B. Weintraube, Schlehdorn, *Cornus sanguinea, Cornus kousa* und *Cornus alba,* konnte keine oder nur eine sehr geringe antibakterielle Wirkung der Kerne festgestellt werden. Diese Ergebnisse zeigen deutlich, dass a priori von einer antibakteriellen Wirkung des Fruchtfleisches nicht auf eine mögliche Wirkung der Fruchtkerne geschlossen werden kann.

Mit dem erfindungsgemäßen Zusatzstoff gelingt es aufgrund der anti-inflammatorischen Wirkung weiterhin, Entzündungsreaktionen zu lindern. Der Zusatzstoff besitzt eine anti-invasive Wirkung, besonders gegen den Pathogen *Lawsonia intracellularis.* Mit dem Zusatzstoff gelingt es weiterhin, das Wachstum von Kokzidien, besonders *Eimeria sp,* im speziellen *Eimeria tenella* zu hemmen sowie Pathogene, insbesondere pathogene *Escherichia coli* Stämme und Endotoxine zu binden. Weiterhin konnte gezeigt werden, dass der Zusatzstoff außerdem eine antivirale Wirkung, im Besonderen gegen Durchfall verursachende Viren, wie beispielswiese Coronaviren oder Rotaviren besitzt.

Der Zusatzstoff weist überdies eine anti-oxidative Wirkung, bevorzugt in der Stärke von mehr als 20 %, insbesondere von mehr als 40 % relativ zur Referenzsubstanz Trolox auf.

Als Quorum Sensing (QS) wird die chemische Kommunikation von Mikroorganismen in Abhängigkeit von der Zelldichte bezeichnet. QS ist an einer Vielzahl von biologischen Aktivitäten wie Biolumineszenz, Bildung von Biofilmen, Sekretion von Antikörpern, aber im Besonderen auch der Sekretion von Pathogenitätsfaktoren beteiligt. In etlichen pathogenen Mikroorganismen konnte QS in der Literatur nachgewiesen werden und in Zusammenhang mit deren Virulenz gebracht werden. Der erfindungsgemäße Zusatzstoff ist überraschenderweise in der Lage, QS zu reduzieren.

Bei Verwendung des Zusatzstoffs in der Nutztierhaltung, bevorzugt als Futtermittelzusatz oder als Trinkwasserzusatz, bevorzugt bei Schweinen, insbesondere Absetzferkel, gelingt es bereits mit geringen Konzentrationen eine stark gesundheitsfördernde Wirkung zu erzielen. Dies zeigt sich in einer deutlichen Reduktion des Durchfallgeschehens. Als Parameter zur Quantifizierung von Durchfall können Durchfalltage, Durchfallscore oder medikamentöse Behandlungen mit einem entsprechenden Arzneimittel zur Reduktion von Durchfall, wie beispielsweise Enteroxid, herangezogen werden.

Bei Verwendung eines erfindungsgemäßen Zusatzstoffs in der Nutztierhaltung, bevorzugt als Futtermittelzusatz oder als Trinkwasserzusatz bei Schweinen, insbesondere Aufzuchtferkel, oder Geflügel, insbesondere Masthühner, gelingt es weiterhin, die Leistung der Nutztiere zu steigern. Die Leistungssteigerung kann anhand gängiger Leistungsparameter, wie beispielsweise Lebendgewicht, Gewichtszunahme, insbesondere tägliche Gewichtszunahme, Futteraufnahme, insbesondere tägliche Futteraufnahme oder Futterverwertungsrate gezeigt werden.

Gemäß einer Weiterbildung der Erfindung ist die in dem Zusatzstoff eingesetzte Kornelkirschenart wenigstens eine, gewählt aus *Cornus mas* und *Cornus officinalis,* welche eine besonders gute antibakterielle und auch antivirale Wirksamkeit zeigen.

Indem der Zusatzstoff so ausgebildet ist, dass das Kornelkirschen-Kernmehl Korngrößen von 50 $\mu$m bis 5 mm, bevorzugt von 100 $\mu$m bis 2 mm aufweist, wird eine Steigerung der antibakteriellen Wirkung erreicht, wobei das Kornelkirschen-Kernmehl in einer Konzentration von bis zu 20 kg/t Futtermittel eingesetzt ist, bevorzugt in einem Bereich von 0,1 kg/t bis zu 10 kg/t Futtermittel, insbesondere bevorzugt in einem Bereich von 0,5 kg/t bis 5 kg/t Futtermittel. Kornelkirschen-Kernmehl kann durch Vermahlen von von Fruchtfleisch befreiten und auf eine Restfeuchte unter 20 Gew.-% getrockneten Kernen hergestellt werden. Durch Trocknung der Kerne auf einen Restfeuchtegehalt von unter 20 Gew.-%, insbesondere unter 13 Gew.-%, sind die Kerne lagerbar und vor mikrobiellem Verderb geschützt. Die Mahlung der Kerne kann mit handelsüblichen Mühlen, wie z.B Schneidmühlen mit entsprechenden Siebeinsätzen im Labor- oder industriellen Maßstab durchgeführt werden.

Gemäß einer Weiterbildung der Erfindung ist der Zusatzstoff so ausgebildet, dass der Kornelkirschen-Kernextrakt ein wässriger, ein organischer, ein wässrig/organischer Kornelkirschen-Kernextrakt oder ein daraus hergestellter Kornelkirschen-Kerntrockenextrakt ist. Als Extraktionsmittel können z.B. Alkohol-Wasser-Mischungen, wie Ethanol-Wasser-mischungen, oder wässrige Pufferlösungen, wie eine phosphatgepufferter Kochsalzlösung, oder Nährmedium für Mikroorganismen oder Zellkultur, oder überkritisches Kohlendioxid, oder sonstige Extraktionsmittel als auch Mischungen davon eingesetzt werden. Zur Herstellung eines Extrakts kann Kornelkirschen-Kernmehl in jedem physikalisch sinnvollen Verhältnis mit einem Extraktionsmittel gemischt werden, bevorzugt in einem Volumensverhältnis Kernmehl zu Extraktionsmittel von 1:4 bis 1:10. Es können auch engere oder weitere Verhältnisse zwischen Extraktionsgut und Extraktionsmittel verwendet werden. Durch Entfernung der Festsubstanzen durch beispielsweise Filtration, Zentrifugation, Separation oder Sedimentation kann ein klarer Extrakt gewonnen werden.

Indem der Zusatzstoff so ausgebildet ist, dass ein Kornelkirschen-Kernextrakt enthalten ist, der mit einem alkoholischen Extraktionsmittel erhalten wurde, wobei als Extraktionsmittel ein Alkohol-Wasser-Gemisch mit mindestens 30 Vol.-% Ethanol, vorzugsweise mit mindestens 50 Vol.-% verwendet wird, gelingt es die antibakterielle Wirkung und die QSinhibierende Wirkung weiter zu erhöhen.

Eine besonders günstige Zusammensetzung des Extrakts wird dadurch erreicht, dass Kornelkirschen-Kernextrakt durch Extraktion bei einer Extraktionstemperatur in dem Bereich von 0 °C bis zum Siedepunkt des Extraktionsmittels, beziehungsweise Extraktionsgemisches, bevorzugt in dem Bereich von 25 °C bis 60 °C, wobei durch Erhöhung der Temperatur die Extraktionsausbeute erhöht wird, erhalten wurde. Die Extraktionsausbeute ist hier als Gewicht von Trockenextrakt bezogen auf das eingesetzte Gewicht des Extraktionsguts definiert und ist in Gewichtsprozent angegeben.

Gemäß einer Weiterbildung enthält der Kornelkirschen-Kernextrakt pro Kilogramm zwischen 70 mg und 7000 mg, bevorzugt zwischen 350 mg und 1500 mg Gallussäure und/oder zwischen 600 mg und 100000 mg, bevorzugt zwischen 2500 mg und 20000 mg Ellagsäure und/oder zwischen 50 mg und 5000 mg, bevorzugt zwischen 250 mg und 1000 mg Loganin und/oder zwischen 1 mg und 2000 mg, bevorzugt zwischen 5 mg und 350 mg Dihydroquercentin.

Der Kornelkirschen-Kernextrakt kann in einer Konzentration von bis zu 10 kg/t Futtermittel eingesetzt werden, bevorzugt in einem Bereich von 2 g/t bis zu 5 kg/t Futtermittel, insbesondere bevorzugt in einem Bereich von 10 g/t bis 1 kg/t Futtermittel.

Durch weitere Anreicherung und Konzentrierung aktiver Komponenten aus Kornelkirschen-Kernextrakten kann ein aufgereinigter Kornelkirschen-Kernextrakt hergestellt werden. Dieser kann beispielsweise mittels chromatographischem Verfahren, wie Säulenchromatographie, oder auch weiteren Extraktionsschritten mit anderen Extraktionsmitteln erfolgen. Der aufgereinigte Kornelkirschen-Kernextrakt kann in einer Konzentration von bis zu 5 kg/t Futtermittel eingesetzt werden, bevorzugt in einem Bereich von 1 g/t bis zu 2,5 kg/t Futtermittel, insbesondere bevorzugt in einem Bereich von 5 g/t bis 500 g/t Futtermittel.

Weiters können jegliche Kornelkirschen-Kernextrakte zu Trockenextrakten weiterverarbeitet werden. Diese können z.B. durch Evaporation der Extraktionsmittel, durch Gefriertrocknung oder jede andere Trocknung hergestellt werden. In der Regel entsteht ein rieselfähiges Pulver, welches mit Fließhilfsstoffen versetzt werden kann. Der Kornelkirschen-Kerntrockenextrakt wird in einer Konzentration von bis zu 10 kg/t Futtermittel eingesetzt werden, bevorzugt in einem Bereich von 1 g/t bis zu 5 kg/t Futtermittel, insbesondere bevorzugt in einem Bereich von 5 g/t bis 1 kg/t Futtermittel. Bei der Verwendung des Extrakts als Futtermittel bzw. Futtermittelzusatzstoff ist es wichtig, dass gegebenenfalls für das Tier toxische Extraktionsmittel vor Verabreichung möglichst vollständig entfernt werden, um deren Rückstände unter für das Tier toxische Konzentrationen zu bringen, um eine Schädigung des Tiers durch z.B. Rückstände von Methanol zu verhindern.

Zur Herstellung von aktiviertem Kornelkirschen-Kernmehl wird der Kornelkirschen-Kernextrakt oder der aufgereinigte Kornelkirschen-Kernextrakt mit einem ausgelaugten Extraktionsgut versetzt und zum Trocknen gebracht.

In einer bevorzugten Weiterbildung der Erfindung wird das aktivierte Kornelkirschen-Kernmehl in einer Konzentration von bis zu 10 kg/t Futtermittel eingesetzt, bevorzugt in einem Bereich von 1 g/t bis zu 5 kg/t Futtermittel, insbesondere bevorzugt in einem Bereich von 5 g/t bis 1 kg/t Futtermittel.

Ein erfindungsgemäßer Zusatzstoff kann weiters hergestellt werden, indem als Ausgangsmaterialen zur Gewinnung von Kornelkirschen-Kernmehl oder Kornelkirschen-Kernextrakt neben Kornelkirschen-Kernen auch Kornelkirschen-Kern-Bruchstücke, Mischungen von Kornelkirschen-Kernen unterschiedlicher Kornelkirschen-Spezies und/oder Mischungen von Kornelkirschen-Kernen mit Kernen anderer Pflanzenspezies eingesetzt werden. Weiterhin können als Ausgangsmaterialen zur Gewinnung von Kornelkirschen-Kernextrakt neben Kornelkirschen-Kernmehl, Mischungen von Kornelkirschen-Kernmehl unterschiedlicher Kornelkirschen-Spezies und/oder Mischungen von Kornelkirschen-Kernmehl mit Kernmehl anderer Pflanzenspezies eingesetzt werden.

Gemäß einer bevorzugten Weiterbildung der Erfindung ist der Zusatzstoff so ausgebildet, dass wenigstens ein weiterer Bestandteil, wie ein Formulierungshilfsstoff, wie ein weiterer Pflanzenbestandteil aus wenigstens einer Kornelkirschen Spezies, gewählt aus der Gruppe Fruchtfleisch, Blätter, Rinde, Stamm, Blüten und Wurzeln und/oder als Trägermaterial, ein physiologisch verträgliches Trägermaterial, vorzugsweise Maltodextrin, Cyclodextrin, Kalziumcarbonat, Stärke, Dinatriumsulfat, Talkum, Saccharose, Bentonite und/oder Zeolithe; eine Futterkomponente wie Getreide; Nebenprodukte aus der Getreideverarbeitung wie Trockenschlempe; Mais, Weizen, Weizenkleie, Reis, Reiskleie, Silage, Öle oder Fette aus pflanzlichen oder tierischen Quellen, Mineralien und/oder Vitamine und/oder eine biologisch wirksame Komponente, gewählt aus der Gruppe Probiotika, Prebiotika, Futtermittelenzyme, Hefen, Hefebestandteile, Säuren, vorzugsweise organische Säuren oder Salze davon; Phytogene; immunstimulierende oder bioaktive Pflanzeninhaltsstoffe und Mykotoxin-deaktivierende Substanzen enthalten ist. Der Formulierungshilfsstoff kann in roher oder verarbeiteter Form, zum Beispiel als Extrakt, eingemischt werden. Mit einem derartigen Zusatzstoff gelingt es, den allgemeinen Gesundheitszustand der Nutztiere weiter zu verbessern, sowie alle Komponenten homogen zu verteilen. Durch Einsatz von wenigstens einer biologisch wirksamen Komponente gelingt es, die Darmflora positiv zu beeinflussen und die Neigung zu Durchfallerkrankungen weiter zu reduzieren.

Gemäß einer Weiterbildung der Erfindung ist der Zusatzstoff so weitergebildet, dass wenigstens ein weiteres Mittel zur Prophylaxe und/oder Behandlung von Durchfallerkrankungen und/oder Verdauungsstörungen enthalten ist. Hierdurch kann die Neigung zu Durchfallerkrankungen und/oder Verdauungsstörungen, hervorgerufen durch einen der folgenden Erreger, *Salmonella sp.,* pathogene *Escherichia coli* Stämme, *Campylobacter sp., Clostridium perfringens, Streptococcus sp., Lawsonia sp., Eimeria sp.* oder pathogene zu QS befähigte Mikroorganismen wie *Yersinia enterocolitica, Vibrio cholerae, Staphylococcus aureus, Candida albicans* oder *Pseudomonas aeruginosa,* Viren wie Rota-oder Coronaviren oder Endotoxinen weiter verringert werden. Der Zusatzstoff kann sowohl in der Nutztierhaltung als auch in der Humanmedizin eine gleichermaßen positive Wirkung zeigen.

Gemäß einer Weiterbildung der Erfindung ist der Zusatzstoff so ausgebildet, dass das weitere Mittel zur Prophylaxe und/oder Behandlung von Durchfallerkrankungen und/oder Verdauungsstörungen aus der Gruppe Antibiotika, Kokzidiostatika, Enteroxid und/oder Zinkoxid gewählt ist. Mit einem derartigen Zusatzstoff gelingt es, Störungen des Magen-Darm-Trakts mit geringen Einsatzmengen des Zusatzstoffes vorzubeugen und/oder zu reduzieren.

Gemäß einer Weiterbildung der Erfindung wird Kornelkirschen-Kernmehl und/oder Kornelkirschen-Kernextrakt aus wenigstens einer Kornelkirschen Spezies, insbesondere *Cornus mas, Cornus officinalis, Cornus chinensis* oder *Cornus eydeana,* sowie zusätzlich wenigstens eine weitere aus chemischer Synthese und/oder mikrobieller Fermentation erhaltene Substanz, gewählt aus Kalziumformiat, Benzoesäure, Ascorbinsäure, Ameisensäure oder Vitamin B12 sowie gegebenenfalls wenigstens ein Bestandteil, gewählt aus Trägermaterialien, Formulierungshilfsstoffen und/oder biologisch wirksamen Komponenten zur Verwendung zur Prophylaxe und/oder Behandlung von Durchfallerkrankungen, insbesondere in der Nutztierhaltung, zur Verfügung gestellt.

Die Erfindung wird nachfolgend anhand vom in der Zeichnung dargestellten Beispiel sowie von Ausführungsbeispielen näher erläutert. In diesen zeigt:

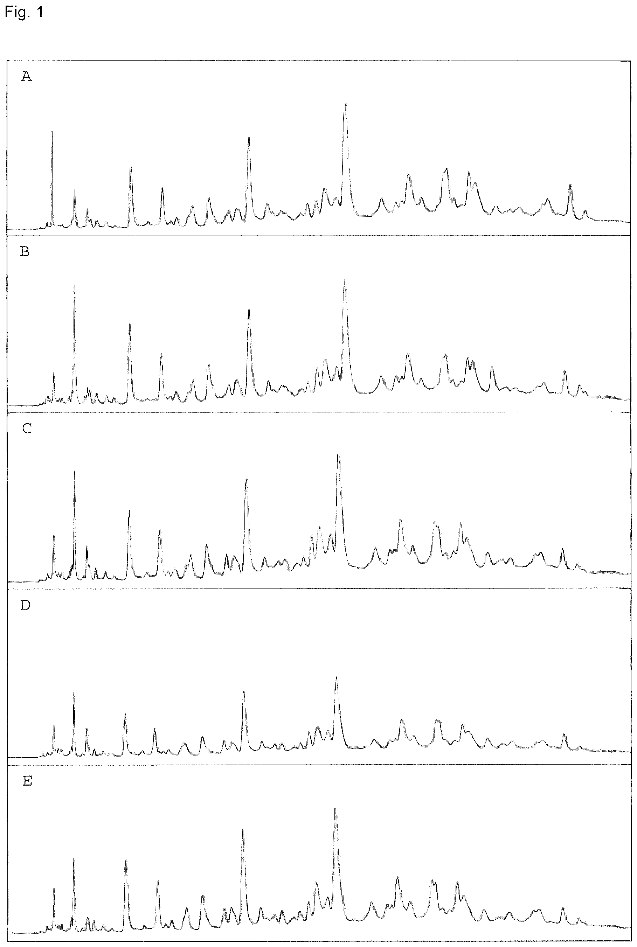

Figur 1: HPLC-DAD Chromatogramme von KKTE von C. mas und C. officinalis verschiedener Herkünfte. A: C. *mas* aus Österreich 1, B: C. *mas* aus Österreich 2, C: C. *mas* aus Türkei, D: C. *mas* aus Deutschland und E: C. *officinalis* aus Deutschland

Beispiel 1: Herstellung von Kornelkirschen-Kernmehl (KKM), Kornelkirschen-Kernextrakt (KKE), Kornelkirschen-Kerntrockenextrakt (KKTE), aufgereinigtem Kornelkirschen-Kernextrakt (AKKE), aufgereinigtem Kornelkirschen-Kerntrockenextrakt (AKKTE) und aktiviertem Kornelkirschen-Kernmehl (AKKM).

Als Ausgangsmaterial dienten getrocknete Kornelkirschen-Kerne der Spezies C. *mas* (Österreich 2) und *Cornus officinalis* (Deutschland). Diese wurden mittels eines handelsüblichen industriellen Trockners auf einen Restfeuchtegehalt von unter 13 Gew.-% getrocknet.

Durch Zerkleinerung der getrockneten Kornelkirschen-Kerne mittels einer handelsüblichen Schneidmühle (Retsch SM100) wurde KKM hergestellt. Bei der Verwendung eines 1,0 mm Siebeinsatzes wurde die folgende Korngrößenverteilung erhalten: 0,05 % >2000 $\mu$m; 14,46 % 1000-2000 $\mu$m; 56,32 % 500-1000 $\mu$m; 19,21 % 250-500 $\mu$m und 9,96 % 125-250 $\mu$m.

Zur Herstellung eines KKE wurde KKM in einem Extraktionsmittel (EM) im Verhältnis von 1:4 bis 1:10 (1 Volumensteil Extraktionsgut zu 4 bis 10 Volumensteilen EM) aufgenommen. Als EM dienten reines Wasser, phosphatgepufferte Kochsalzlösung (PBS-Puffer: 8,0 g/l Natriumchlorid, 0,2 g/l Kaliumchlorid und 1,42 g/l Dinatriumhydrogenphosphat, pH = 7,4), Nährmedium mit 10 Vol.-% Ethanol sowie Ethanol-Wassermischungen mit Ethanolanteilen von 50, 70 und 96 Vol.-%. Ethanol. Die Extraktion erfolgte durch Schütteln oder Rühren bei 50 U/min bis 100 U/min. Die Extraktionsdauer betrug zwischen 1h und 48 h. Nach der Extraktion wurden die Feststoffanteile mittels Filtration oder Zentrifugation vom Extrakt abgetrennt, sodass ein klarer und gegebenenfalls durch Sterilfiltration steriler Extrakt erhalten wurde.

Der zum Extrahieren eingesetzte Alkohol wurde durch Verdampfung im Rotations-Evaporator aus dem KKE entfernt. Die verbleibende Lösung wurde durch Gefriertrocknung zur Trockene gebracht. Dadurch entstand KKTE als rieselfähiges Pulver.

Der Einfluss der Extraktionsdauer und Extraktionstemperatur, definiert als Temperatur von Extraktionsgut und EM, auf die Extraktionsausbeute wurde anhand einer Extraktion von C. *mas* KKM, das mit einer Schneidmühle mit einem 1,0 mm Siebeinsatz hergestellt wurde, und einer Ethanol-Wassermischung mit 50 Vol.-% Ethanol als EM untersucht. Das Volumensverhältnis von Extrationsgut zu EM betrug 1:6. Die Ausbeute ist definiert als das Gewicht von KKTE bezogen auf das eingesetzte Gewicht des Extraktionsguts und wird in Gew.-% angegeben. Die Extraktionsausbeute betrug 10,36 Gew.-% bei 25 °C, über 10,75 Gew.-% bei 40 °C bis zu 13,13 Gew.-% bei 60 °C jeweils bei 1 h Extraktion und auf 14,06 Gew.-% bei 25 °C, 15,56 Gew.-% bei 40 °C bis zu 17,78 Gew.-% bei 60°C jeweils bei 21 h Extraktion, woraus ersichtlich ist, dass die Ausbeute mit zunehmender Temperatur und Extraktionsdauer zunahm.

Zur Herstellung eines AKKE wurde ein mittels ethanolischer Extraktion erhaltener KKTE aus *C. mas* weiter aufgereinigt. Der KKTE wurde durch Extraktion mit einer 70:30 Ethanol-Wassermischung und anschließender Ethanol-labtrennung im Rotationsverdampfer sowie Gefriertrocknung des verbleibenden Extrakts erhalten. 700 mg des KKTE wurden in 5 ml Heptan aufgenommen und über Säulenchromatographie aufgereinigt. Als Säulenmaterial wurde handelsübliches Kieselgel 60 mit einem Durchmesser von 0,063 mm bis 0,200 mm von der Firma Merck verwendet. Die Säulendimensionen waren 300 x 15 mm. Als Laufmittel wurde ein Stufengradient an reinem Heptan, 50:50 Heptan/Ethylacetat, reinem Ethylacetat, 75:25 Ethylacetat/Methanol, 50:50 Ethylacetat/Methanol, 25:75 Methylacetat/Methanol und reinem Methanol verwendet, wobei nach jeweils 100 ml die Laufmittelzusammensetzung geändert wurde, sodass die Elutionskraft zunahm.

Fraktionen von je 100 ml wurden gesammelt und mittels Rotationsverdampfer zur Trockene gebracht. Fraktion Nummer 4 zeigte von allen Fraktionen die stärkste antibakterielle Wirkung gegen *E. coli* O128:H2. Fraktion Nummer 4 entspricht daher einem AKKE und in getrockneter Form einem AKKTE.

Des Weiteren wurden AKKE bzw. AKKTE durch Flüssig-Flüssig-Extraktion von KKTE hergestellt. 500,52 mg eines KKTE, der durch eine Extraktion mit einer Ethanol-Wassermischung von 70 Vol.-% Ethanol und anschließender Ethanolabtrennung im Rotavapor sowie Gefriertrocknung erhalten wurde, wurde mit 100 ml Wasser und 100 ml Chloroform versetzt und in einem Scheidetrichter ausgeschüttelt. Die wässrige und die organische Phase wurden separiert und durch Evaporation im Rotations-Evaporator zur Trockene gebracht, wobei 392,12 mg Trockenextrakt aus der wässrigen Phase und 108,40 mg Trockenextrakt aus der Chloroformphase erhalten wurden. Ausschließlich die wässrige Phase zeigt eine antibakterielle Wirkung, während die Chloroformphase keine Aktivität zeigte. Der wässrige Extrakt entspricht demnach einem AKKE und der Trockenextrakt davon entspricht einem AKKTE.

Ein AKKM wurde hergestellt, indem 50,23 g KKM von *C. mas,* hergestellt mit einer Schneidmühle mit 1,0 mm Siebeinsatz, mit 200 ml Ethanol 50 Vol.-% unter Schütteln (60 U/min 25°C) für 24 Stunden extrahiert wurde. Anschließend wurde der gewonnene KKE zusammen mit dem ausgelaugten KKM gemischt und im Rotations-Evaporator zur Trockene gebracht, sodass AKKM als ein rieselfähiges Produkt entstand.

Beispiel 2: Antibakterielle Wirkung von Fruchtkernen.

Die Bestimmung der antibakterielle Wirkung der Testsubstanzen wurden mittels Mikroverdünnungs-Hemmtest nach den Richtlinien des "Clinical and Laboratory Standards Institutes" (CLSI Standards M45A, M2 und M7) durchgeführt. Aus der resultierenden Wachstumshemmung der Bakterienstämme wurde die Minimale-Hemm-Konzentration (MHK50), das ist die Konzentration bei der das bakterielle Wachstum um zumindest 50 % gehemmt wird, bestimmt. Die MHK50 dient als Maß für die antibakterielle Wirkung von Substanzen. Je geringer dieser Wert ist, desto stärker ist die antibakterielle Wirkung.

Um die antibakterielle Wirkung von Fruchtkernen von Marille (*Prunus armeniaca*), Kirsche (*Prunus avium*), Weichselkirsche (*Prunus cerasus*), Kriecherl (*Prunus domestica ssp insititia*) und einer Kornelkirschen-Spezies (*Cornus mas*) zu untersuchen, wurden die Kerne mit einer Schneidmühle mit einem 1,0 mm Siebeinsatz zu Kernmehl gemahlen, im Verhältnis 1:5 (v/v) mit einer Ethanol-Wassermischung mit 70 Vol.-% Ethanol für 24 h unter Schütteln (50 U/min) bei 25 °C extrahiert und ein Kerntrockenextrakt durch Gefriertrocknung hergestellt, wie in Beispiel 1 beschrieben. Deren antibakterielle Wirkung wurde gegen den enteropathogenen Bakterienstamm *Escherichia coli* O128:H2 untersucht.

Es wurden unterschiedliche Konzentrationen der Kerntrockenextrakte gemeinsam mit dem Bakterienstamm entsprechend den Vorgaben der Richtlinie des CLSI kultiviert. Der getestete Konzentrationsbereich lag zwischen 39 mg und 1250 mg Kerntrockenextrakt/l Medium. Die antibakterielle Wirkung von Kernextrakten von Marille (*Prunus armeniaca*), Kirsche (*Prunus avium*), Weichselkirsche (*Prunus cerasus*), Kriecherl (*Prunus domestica* ssp insititia) auf den enteropathogenen Bakterienstamm *Escherichia coli* O128:H2 betrug jeweils MHK50 [mg/l] >1250 und jene der Kornelkirsche (*Cornus mas*) betrug MHK50 [mg/l] 78-156. Daraus ist ersichtlich, dass, außer dem KKE aus *Cornus mas*, keiner der Kernextrakte eine antibakterielle Wirkung besitzt.

Des Weiteren wurde die anti-mikrobielle Wirkung von Kernen anderer Cornus Spezies, nämlich *Cornus sanguinea, Cornus kousa* und *Cornus alba* sowie von Kernen anderer Früchte, nämlich Weintrauben, (*Vitis vinifera*) und Schlehdorn (*Prunus spinosa*) untersucht.

Um auch den Einfluss unterschiedlicher Korngrößen des Kernmehls zu untersuchen, wurde Kernmehl von *Cornus mas* in einer Schneidmühle mit drei unterschiedlichen Siebeinsätzen, nämlich 0,5 mm, 1,0 mm und 2,0 mm Durchmesser, hergestellt. Für alle anderen Kerne wurde die Korngröße von 1,0 mm gewählt. Je 1 g Kernmehl wurde in einem 15 ml Zentrifugenröhrchen eingewogen und mit 9 g einer Ethanol-Nährbouillonmischung mit 10 Vol.-% Ethanol versetzt. Die Proben wurden für 1 h unter Schütteln auf einem Horizontalschüttler (90 U/min) bei 25 °C extrahiert. Anschließend wurde zentrifugiert und der klare Überstand für die Hemmtests verwendet.

Die Hemmtests auf antibakterielle Wirkung wurden mit dem Mikroverdünnungs-Testsystem gegen *E. coli* O128:H2, wie in den CLSI Standards M45A, M2 und M7 beschrieben, durchgeführt. Es wurden die Extrakte mit Nährmedium in den Verdünnungsstufen 1:4, 1:8, 1:16, 1:32, 1:64 und 1:128 hergestellt und getestet. Als Indikator für die antibakterielle Wirkung wurde die höchste Verdünnungsstufe herangezogen, die zu mindestens 50 % Hemmung des Bakterienwachstums im Vergleich zu einer Ethanol-Nährbouillonmischung ohne Testsubstanz (Negativkontrolle) führte. Je höher die Verdünnungsstufe ist, mit der eine 50 % Wachstumshemmung erreicht werden kann, desto stärker ist die anti-mikrobielle Wirkung der Testsubstanz.

Als Ergebnis zeigte sich, dass die Kernextrakte von *Cornus mas* in der Lage waren, selbst bei einer Verdünnung von 1:64 und 1:128 das Wachstum von *E. coli* O128:H2 um mindestens 50 % zu hemmen. Bei den andern Kernextrakten war selbst in der niedrigsten Verdünnungsstufe, nämlich 1:4, keine Hemmung des Bakterienwachstums detektierbar.

Beispiel 3: Antibakterielle Wirkung von Kornelkirschen-Kernen im Vergleich zu Kornelkirschen-Fruchtfleisch und Kornelkirschen-Destillationsrückstand.

Die Bestimmung der antibakterielle Wirkung der Testsubstanzen wurden mittels Mikroverdünnungs-Hemmtest nach Richtlinien des "Clinical and Laboratory Standards Institutes" (CLSI Standards M45A, M2 und M7) durchgeführt.

Um die antibakterielle Wirkung von Kernen, Fruchtfleisch sowie Destillationsrückstand von *C. mas* Früchten vergleichen zu können, wurden Trockenextrakte, wie in Beispiel 1 beschrieben, hergestellt. Die Extraktion erfolgte mit 1:5 (v/v) mit einer Ethanol-Wassermischung mit 70 Vol.-% Ethanol für 24 h unter Schütteln (50 U/min) bei 25 °C. Als Destillationsrückstand diente vergorene und destillierte Maische von *C. mas* Früchten. Die Maische enthielt das Fruchtfleisch und die Schale, jedoch nicht Kerne. Der eingesetzte Konzentrationsbereich von Kern-Trockenextrakt, Fruchtfleisch-Trockenextrakt und Destillationsrückstand betrug 39 mg/l bis 2500 mg/l Medium.

Die Ergebnisse sind in Tabellen 1 und 2 zusammengefasst. Daraus ist ersichtlich, dass der Kernextrakt eine weit höhere antibakterielle Wirkung besitzt als der Fruchtfleischextrakt und der Destillationsrückstand. Der Fruchtfleischextrakt und der Destillationsrückstand haben nahezu keine antibakterielle Wirkung im getesteten Konzentrationsbereich. Das wurde für alle acht getesteten, pathogenen Bakterienstämme festgestellt, unabhängig davon, ob diese Gram-negativ oder Gram-positiv sind.

Tabelle 1: Antibakterielle Wirkung von *C*. mas-Kernextrakt, *C*. mas-Fruchtfleischextrakt und *C*. mas-Destillationsrückstand gegen pathogene Gram-negative Bakterienstämme.

| | Bakterienstamm | | | | |
|---|---|---|---|---|---|
| | *Salmonella typhimurium* | *Salmonella enteritidis* | *E. coli* O128:H2 | *E. coli* O8: K87 F4 | *Campylobacter coli* |
| | Nährmedium | | | | |
| | Nährbouillon | Nährbouillon | Nährbouillon | Trypton-Soja-Brühe | Müller-Hinton-Brühe |
| | MHC50 [mg / L] | | | | |
| Destillationsrückstand | 1250/2500 | >2500 | >2500 | 2500 / >2500 | 2500 / >2500 |
| Fruchtfleischextrakt | 2500 | >2500 | 2500 / >2500 | >2500 | >2500 |
| Kernextrakt | 39/78 | 39 | 78 / 156 | 313 | 39 / 156 |

Tabelle 2: Antibakterielle Wirkung von *C*. mas-Kernextrakt, *C. mas*-Fruchtfleischextrakt und *C. mas*-Destillationsrückstand gegen pathogene Gram-positive Bakterienstämme.

| | Bakterienstamm | | |
|---|---|---|---|
| | *Clostridium perfringens Toxin Typ C* | *Clostridium perfringens Toxin Typ A* | *Streptococcus suis* |
| | Nährmedium | | |
| | Clostridien-Medium | Clostridien-Medium | Hirn-Herz-Brühe |
| | MHC50 [mg / L] | | |
| Destillationsrückstand | 1250 / >2500 | 2500 / >2500 | 2500 |
| Fruchtfleischextrakt | >2500 | >2500 | 2500 |
| Kernextrakt | 313 | 156 | 313 |

Beispiel 4: Einfluss von Kornelkirschen-Spezies, von Kornelkirschen-Anbaugebieten, von unterschiedlichen Extraktionstemperaturen und von unterschiedlichen Extraktionsmitteln auf die antibakterielle Wirkung von Kornelkirschen-Kernen.

Der Einfluss unterschiedlicher Kornelkirsche-Spezies, nämlich *Cornus mas* und *Cornus officinalis,* sowie unterschiedlicher Extraktionstemperaturen (25 °C und 60 °C) und unterschiedlicher Anbaugebiete (Österreich 1 und 2, Türkei und Deutschland) auf die antibakterielle Wirkung der Kerne wurde mittels einem Mikroverdünnungs-Hemmtest nach den Richtlinien des "Clinical and Laboratory Standards Institutes" (CLSI Standards M45A, M2 und M7) getestet.

Für alle Tests wurde Kernmehl eingesetzt, welches durch Zerkleinerung, wie in Beispiel 1 beschrieben, hergestellt wurde. Die Extrakte wurden im Verhältnis 1:5 (v/v) entweder mit Wasser, oder mit Ethanol-Wassermischung mit 50 Vol.-%, 70 Vol.-% oder 96 Vol.-% Ethanol hergestellt, wobei für 24 h unter Schütteln (50 U/min) bei 25 °C extrahiert wurde. Die Extrakte wurden, wie in Beispiel 1 beschrieben, zu Trockenextrakten weiterverarbeitet und getestet.

Die Ergebnisse der unterschiedlichen Kornelkirschen-Spezies, Extraktionstemperaturen sowie Anbaugebiete zeigen, dass die Verwendung unterschiedlicher Kornelkirschen-Spezies, sowie die Herkunft des Rohmaterials als auch die unterschiedlichen Extraktionstemperaturen keinen wesentlichen Einfluss auf die antibakterielle Wirkung der Kernextrakte gegen *E. coli* O128:H2 in Nährbouillon hatten. Die MHK50 [mg/l] waren 78 - 156 bei C. *mas* aus Österreich 1, 156 bei C. *mas* aus Österreich 2, 156 - 313 bei C. *mas* aus Türkei, 156 - 313 bei *C. mas* aus Deutschland und 156 - 313 bei *C. officinalis* aus Deutschland.

Die Ergebnisse der unterschiedlichen Extraktionsmittel auf die antibakterielle Wirkung der Extrakte zeigen, dass das Extraktionsmittel einen Einfluss auf die antibakterielle Wirkung der Kernextrakte besitzt, wie dies in Tabelle 3 gezeigt ist. Der 50 Vol.-%ige Ethanol-Extrakt wirkt gegen *E. coli* O128:H2 und S. *typhimurium* am stärksten, gegen *C. perfringens* Typ A wirkt er gleich stark wie der Wasserextrakt. Die antibakteriellen Wirkungen des Wasserextrakts, des 70 Vol.-%igen und des 96 Vol.-%igen Ethanol-Extrakts sind deutlich ersichtlich, jedoch schwächer ausgeprägt als die des 50 Vol.-%igen Ethanol-Extrakts.

Tabelle 3: Antibakterielle Wirkung verschiedener von *C. mas* Kern-Trockenextrakte gegen pathogene Keime. Die Extraktionstemperatur betrug 25 °C. Anbaugebiet von *C. mas* war Österreich 1.

| Extraktionsmittel | MHK50 [mg / l] | | |
| --- | --- | --- | --- |
| | *E. coli* O128:H2 | *S. typhimurium* | *C. perfringens* Toxin Typ A |
| 100 % Wasser | 156 - 313 | 78 - 156 | 313 |
| 50 Vol.-% Ethanol | 78 - 156 | 39 | 313 |
| 70 Vol.-% Ethanol | 78 - 156 | 39 - 78 | 313 |
| 96 Vol.-% Ethanol | 156 - 313 | 78 | 625 |

*C. mas* Kern-Trockenextrakte zeigen des Weiteren eine antibakterielle Wirkung gegen eine Vielzahl von Bakterienstämmen. Besonders gut wurde das Wachstum pathogener Stämme, wie die *S. enteritidis* (MHK50 [mg/l] = 39), *S. typhimurium* (MHK50 [mg/l] = 39 - 78), *C. coli* O128:H2 (MHK50 [mg/l] = 78 - 156) und *Campylobacter coli* (MHK50 [mg/l] = 39 - 156) inhibiert. Im Gegensatz dazu wurden die probiotischen Bakterien, wie *E. faecium* und *L. salivarius* erst mit vergleichsweise sehr hohen Konzentrationen, mit MHK50 Werten zwischen 625 und 1250 mg/l, im Wachstum gehemmt.

Beispiel 5: Anti-inflammatorische Wirkung von Kornelkirschen-Kernen.

Die anti-inflammatorische Wirkung von Kornelkirschen-Kernen wurde mit Lipopolysaccharid (LPS) stimulierten Mausmakrophagen getestet. Der Kontakt mit LPS aus pathogenen Bakterien führt zur Aktivierung der Entzündungsreaktion von Makrophagen, was die Sekretion von Stickstoffmonoxid (NO) zur Folge hat. Durch die Zugabe von KKTE konnte dessen anti-inflammatorische Wirkung, nämlich über die Abnahme der NO-Sekretion durch die mit LPS stimulierten Zellen, gezeigt werden.

Als Testmaterial diente ein wie in Beispiel 1 beschriebener KKTE von *C. mas.*

Zur Bestimmung der anti-inflammatorischen Wirkung wurden Makrophagen (Raw 264.7) *in vitro* in 96-well Mikrotiterplatten ausgesät (20000 Zellen pro well). Als Nährmedium wurde Dulbeccos's Modified Eagle's Medium (DMEM) Ham's F12 (1:2), supplementiert mit 2 mM Glutamin und 10 Vol.-% Fötales Kälberserum (FKS) verwendet. Zur Stimulierung der Entzündungsreaktion wurden die Zellen mit 10 ng/ml LPS von *Escherichia coli* O111:B4 (Sigma L2630) versetzt und 24 h bei 37 °C, 5 % $CO_2$ und 95 % relativer Luftfeuchtigkeit inkubiert. Das bei der Simulation gebildete NO reagiert innerhalb von wenigen Sekunden mit Sauerstoff zu Nitrit ($NO_2^-$). $NO_2^-$ wurde mit einem Standardverfahren mit Hilfe des Griess-Reagenz (Sigma G4410) photometrisch bei 540 nm quantifiziert. Die NO-Produktion wurde als "Relativer NO-Gehalt %" dargestellt. Als Positivkontrolle dienten Zellen, die mit 10 ng/ml LPS stimuliert wurden. Als Negativkontrolle diente eine reine Zellkontrolle der keine Testsubstanz zugesetzt wurde. Der gemessene relative NO-Gehalt der Positivkontrolle wurde als 100,00 %, der Negativkontrolle als 0,00 % definiert. Der KKTE [50 $\mu$g/ml] führte zu keiner wesentlichen NO-Produktion mit einem relativen NO-Gehalt von 1,32 %. Durch die Zugabe des KKTE [50 $\mu$g/ml] zusammen mit LPS konnte die NO-Produktion von 100,00 % auf 80,60 %, das heißt um 19,40 Prozentpunkte, reduziert werden. Die Reduktion des NO-Gehalts kann als Maß für die anti-inflammatorische Wirkung von Substanzen angesehen werden. Somit konnte eine deutliche anti-inflammatorische Wirkung von Kornelkirschen-Kernen gezeigt werden.

Beispiel 6: Anti-invasive Wirkung von Kornelkirschen-Kernen.

Die anti-invasive Wirkung von Kornelkirschen-Kernen wurde anhand eines *Lawsonia intracellularis*-Invasionstests gezeigt.

Als Testmaterial diente ein, wie in Beispiel 1 beschriebener, hergestellter Kornelkirschen-Kerntrockenextrakt von *Cornus mas.* Für den Invasionstest wurde der Trockenextrakt in 70 % Ethanol gelöst, mit einer phosphatgepufferten Salzlösung (PBS-Puffer: Zusammensetzung: 8,0 g/l Natrimchlorid, 0,2 g/l Kaliumchlorid und 1,42 g/l Dinatriumhydrogenphosphat, pH = 7,4) weiter verdünnt und durch einem 0,2 $\mu$m Sterilfilter filtriert.

*L. intracellularis* wurde in Form eines Lebendimpfstoffs für orale Verabreichung nämlich Enterisol Ileitis von Boehringer Ingelheim bezogen. Der Impfstoff wurde in PBS-Puffer aufgenommen und sequenziell mit einem 5,0 $\mu$m und 0,8 $\mu$m Filter von nicht löslichen Fragmenten befreit, um eine reine Bakteriensuspension ($10^5$ Bakterien pro ml) zu erhalten.

Aliquote der *L. intracellularis* Suspension wurden zu einer Verdünnungsreihe der C. *mas* Lösung hinzugegeben (jeweils 100 $\mu$l Suspension und 100 $\mu$L Probelösung in 96-well-Mikrotiterplatten) und für 30 min bei 37 °C, 8,8 % $CO_2$

und 8 % O$_2$ inkubiert. Zusätzlich wurden *L. intracellularis* in reinem PBS-Puffer und in 70 % Propanol als Lebend- und Totkontrollen inkubiert.

Die Bakterien wurden anschließend mit dem LIVE/DEAD BacLight Bacterial Viability Kit (Invitrogen, L34856) laut Anleitung gefärbt und mit einem ACCURI C6 Durchflusszytometer analysiert. Tote und lebende *L. intracellularis* konnten anhand ihrer Fluoreszenz unterschieden werden. Die Viabilität der mit C. *mas* inkubierten *L. intracellularis* Suspensionen wurde mit der Viabilität der Lebendkontrolle verglichen, um die relative Viabilität abzuleiten. Die relative Viabilität nimmt mit steigenden C. mas-Kernextrakt Konzentrationen wie folgt ab: 93 % bei einer Konzentration von 3,12 mg/l, 89 % bei einer Konzentration von 6,25 mg/l, 80 % bei einer Konzentration von 12,50 mg/l, 56 % bei einer Konzentration von 25 mg/l, 32 % bei einer Konzentration von 50 mg/l und 15 % bei einer Konzentration von 100 mg/l. Die MHK50 liegt zwischen 25 und 50 mg/l. Dadurch kann einer antibakteriellen und in weiterer Folge auch eine anti-invasive Wirkung von *Cornus mas* Kernen gegen *L. intracellularis* gezeigt werden.

Beispiel 7: Anti-kokzidielle Wirkung von Kornelkirschen-Kernen

Die anti-kokzidielle Wirkung von Kornelkirschen-Kernen wurde mittels Hemmung der Invasion des intrazellulären Parasiten *Eimeria tenella* durch C. mas-Kernextrakt in Zellkultur bestimmt.

Als Testmaterial wird ein wie in Beispiel 1 beschriebener Kornelkirschen-Kerntrockenextrakt von *Cornus mas* verwendet. Dieser Trockenextrakt wurde für die jeweiligen Hemmtests in 70 % Ethanol gelöst und mit Zellkulturmedium verdünnt.

Oozysten von *E. tenella* wurden vom Royal Veterinary Institute (London, GB) bezogen und bei 4 °C gelagert, Kurz vor dem Experiment wurden Sporozoiten aus den Oozysten aufgereinigt (Mattig et al.; Appl, Parasität, 1993, 34:139-142) und mit dem Fluoreszenzfarbstoff CFDA-SE markiert (Schubert et al.; Parasität Res., 2005, 197:59-62).

MDBK Zellen (ECACC=Nr. 90050801) wurden in 96-well Mikrotiterplatten ausgesät (50000 Zellen pro well) und nach 24 h mit den fluoreszenten *E. tenella* Sporozoiten infiziert. Die infizierten Zellen wurden über Nacht entweder in verschiedenen Konzentrationen des Testmaterials oder reinem Zellkulturmedium, als Kontrolle, bei 40 °C und 5 % CO$_2$ und 95 % relativer Luftfeuchtigkeit inkubiert. Während dieser Inkubationszeit drangen Sporozoiten in die MDBK Zellen ein. Nach der Inkubation wurde die Viabilität der MDBK Zellen mit einem Tetrazoliumsalztest (WST-1, Roche, #11644807001) überprüft. Die relative Zellviabilität wurde im Vergleich zur Kontrolle ermittelt. Danach wurden entweder mikroskopisch oder mit einem Durchflusszytometer die fluoreszenten intrazellulären Sporozoiten gezählt, um die Invasionsraten von Kontrolle und Test zu vergleichen. Für jeden Test wurde eine Hemmrate nach folgender Formel berechnet:

*Hemmung der Zellinvasion [%] =*

*1 – (Invasionsrate eines Testwells/ durchschnittliche Invasionsrate der Kontrollwells)*

Die Ergebnisse des Invasionshemmtests sind in Tabelle 4 zusammengefasst und zeigen eine deutliche anti-kokzidielle Wirkung des C. mas-Kerntrockenextrakts mit einer MHC50 von 62,5 mg/l. Die Viabilität der Wirtszellen wurde durch die Zugabe des Trockenextrakts nicht negativ beeinflusst.

Tabelle 4: *E. tenella* Invasionshemmung durch C. mas-Kerntrockenextrakt in MDBK Zellen.

| Konzentration [mg/l] | Relative Hemmung | Relative Standardabweichung der Hemmung | Relative Viabilität | Relative Standardabweichung der Viabilität |
|---|---|---|---|---|
| 62,50 | 52 % | 16 % | 103 % | 11 % |
| 15,63 | 14 % | 23 % | 110 % | 8 % |
| 3,91 | 8 % | 20 % | 112 % | 2 % |

Beispiel 8: Pathogenbindende Wirkung von Kornelkirschen-Kernen

Die pathogenbindende Wirkung von Kornelkirschen-Kernen wurde mittels Bindungstest von dem Gram-negativen, enterotoxischen Bakterien des Stammes *Escherichia coli* O8:K87 (F4) gezeigt.

Als Testmaterial dienten *C. mas* Kernmehl, dass durch Zerkleinerung von Kernen mit einer Schneidmühle und einem Siebeinsatz von 1,0 mm hergestellt wurde. Daraus wurde eine 1 Gew.-%ige sowie eine 0,1 Gew.-%ige Suspension in phosphatgepufferter Kochsalzlösung (PBS-Puffer: Zusammensetzung: 8,0 g/l Natrimchlorid, 0,2 g/l Kaliumchlorid und 1,42 g/l Dinatriumhydrogenphosphat, pH = 7,4) hergestellt. 100 µl dieser Suspensionen wurden in einer Mikrotiterplatte über Nacht bei 4 °C inkubiert. Als Positivkontrolle diente eine Suspension aus Zellwandbestandteilen von *Saccharomyces*

*cerevisiae* mit bekannt guter Bindungskapazität. Der Versuch wurde, wie in Ganner et al.; Micobiology Methods; 2010, 83(2), 168 ff. beschrieben, durchgeführt.

Die Ergebnisse zeigen eine deutliche und konzentrationsabhängige Bindung des pathogenen *E. coli* O8:K87 (F4) durch die eingesetzte *C. mas*-Kernmehlsuspension. Die Zahl der gebundenen Keime wird als koloniebildende Einheit (KBE) angegeben. Die 1 Gew.-%ige *C. mas*-Kernmehlsuspension war in ihrer Bindungsstärke von 463,6 KBE/ml vergleichbar mit der 1 Gew.-%igen Hefezellwandsuspension (607 KBE/ml). Bei einer 10-fach geringeren Konzentration an *C. mas* Kernmehl war eine um etwa 10-fach geringere Pathogenbindung von 36,9 KBE/ml feststellbar.

Diese gute Bindung von pathogenen Keimen durch *C. mas*-Kernmehl hat eine günstige Wirkung bei Durchfallerkrankungen, die normalerweise von diesen Bakterien ausgelöst werden. Die pathogenen Keime binden anstatt an das Darmepithel an die Produktmatrix und werden ausgeschieden, ohne ihre pathogene Wirkung zu entfalten.

Beispiel 9: Endotoxin-neutralisierende Wirkung von Kornelkirschen-Kernen.

Die endotoxin-neutralisierende Wirkung von Kornelkirschen-Kernen wurde mittels Limulus-Amöbocyten-Lysat (LAL-Test, Endochrome K, Charles River Laboratories, Inc. Charleston, USA - R1708K) festgestellt.

Als Testmaterial diente C. mas-Kernmehl mit einer dominierenden Korngröße von 0,5 - 2 mm (78 % der Masse). Aus diesem wurden mit pyrogenfreiem Wasser (PFW) oder mit künstlicher Darmflüssigkeit (KDF, US Pharmacopeia 25) Kernmehlsuspensionen mit unterschiedlichen Konzentrationen, nämlich 0,1 Gew.-%, 0,05 Gew.-% und 0,01 Gew.-% Kernmehl, hergestellt. Die Suspensionen wurden mit unterschiedlichen Mengen Lipopolysacchariden (LPS) aus einer Endotoxinstammlösung von *E. coli 055:B5* (Sigma Aldrich, L2880) mit einer Konzentration von 500.000 Endotoxin Units pro Milliliter (EU/ml) versetzt, sodass die finale EU-Konzentration in der Suspension 4.000 EU/ml, beziehungsweise 10.000 EU/ml betrug. Das *C. mas*-Kernmehlsuspension - LPS Gemisch wurde für 2 h bei 37 °C und 1000 U/min geschüttelt.

Anschließend wurden die Proben hitzeinaktiviert (15 min, 70 °C) und abzentrifugiert (500 RCF für 30 min). Der Überstand wurde für den LAL-Test verwendet. Der LAL-Test wurde entsprechend den Herstellerangaben durchgeführt. Der Überstand wurde verdünnt und in eine endotoxinfreie 96-well Mikrotiterplatte überführt, anschließend wurde der verdünnte Überstand mit einer definierten Endotoxinlösung (10 EU/ml) versetzt (gespikt). Die Zugabe der Endotoxinlösung diente der Bestimmung der Wiederfindungsrate. Eine entsprechende Wiederfindungsrate (50 - 200 %) diente als Qualitäts- und Ausschlusskriterium für jede Analyse. Anschließend wurde das LAL-Reagenz hinzugefügt, und die Platte bei 37 °C für 70 min im vorgewärmten Plate Reader bei 405 nm gemessen. Die Berechnung des Neutralisationsgrads erfolgt über die Software EndoScan-V 8 von Charles River.

Im untersuchten Konzentrationsbereich von 0,01 Gew.-% bis 0,1 Gew.-% zeigte *C. mas*-Kernmehl eine sehr gute Endotoxin-neutralisierende Wirkung. Im pyrogenfreien Wasser und einer LPS Konzentration von 10000 EU/ml konnten mit einer 0,01 Gew.-%igen Kernmehlsuspension 34,45 %, mit einer 0,05 Gew.-%igen Kernmehlsuspension 26,34 % des LPS neutralisiert werden. In der komplexen Matrix des künstlichen Darmsafts und einer LPS Konzentration von 4000 EU/ml konnten mit einer 0,01 Gew.-%igen Kernmehlsuspension 33,77 %, mit einer 0,1 Gew.-%igen Kernmehlsuspension 27,12 % des LPS neutralisiert werden.

Beispiel 10: Anti-oxidative Wirkung von Kornelkirschen-Kernen.

Die anti-oxidative Wirkung von Kornelkirschen-Kernen wurde mit Hilfe des ORAC (Oxygen Radical Antioxidant Capacity) Tests untersucht (ZenBio Inc., USA, NC, #AOX-2). Der ORAC Test misst den Wasserstoffatom-Transfer zwischen reaktiven Sauerstoffspezies (reactive oxygen species, ROS) und dem Testmaterial.

Als Testmaterial diente ein, wie in Beispiel 1 beschrieben, hergestellter Kornelkirschen-Kerntrockenextrakt von *C. mas*. Der Trockenextrakt wurde in 5 verschiedenen Konzentrationen von 1,56 bis 25,00 mg/l getestet.

Der ORAC Test beruht auf der Oxidation des Fluoreszenzfarbstoffs Fluoreszein Natrium. Wird der Farbstoff oxidiert, nimmt seine Fluoreszenzintensität ab. Die Oxidation wurde durch 2,2'-Azobis(2-methylpropion-amidin)dihydrochlorid (ABAP), welches ROS generiert, beschleunigt und die zeitliche Abnahme der Fluoreszenz wurde mit einem Mikroplattenlesegerät gemessen. In der Anwesenheit von Antioxidantien, wie dem *C. mas*-Kernextrakt, wurde die Oxidation gehemmt, was zu einer verzögerten Abnahme der Fluoreszenz führte. Die Bestimmung der anti-oxidativen Wirkung der Testsubstanzen erfolgte über die Kinetik der Fluoreszenzabnahme. Es ergibt sich eine lineare Dosis-Wirkungs-Beziehung und die Steigung der Geraden wird als Maß für die Wirksamkeit herangezogen. Die Ergebnisse werden in Relation zu dem Vitamin E Analogon Trolox (6-Hydroxy-2,5,7,8-tetramethylchroman-2-carbonsäure) dargestellt, welches als Standard mit bekannter anti-oxidativen Kapazität dient. Die anti-oxidativen Wirkungen der Testsubtanzen wurden als Trolox Äquivalente (TE) dargestellt, wobei die Geradensteigung der Testsubstanz mit jener von Trolox (definiert als 1 TE) in Beziehung gesetzt wird.

Ergebnis: Der *C. mas*-Kernextrakt zeigte eine anti-oxidative Kapazität von 0,46 ± 0,05 TE. Das bedeutet, dass der Extrakt circa 46 % der anti-oxidativen Kapazität von reinem Trolox besitzt.

Beispiel 11: Inhibierung von Quorum Sensing durch Kornelkirschen-Kernen.

Die Quorum Sensing (QS) inhibierende Wirkung von Kornelkirschen-Kernen wurde mittels Biosensoren, das sind bekannte Indikatorstämme wie Chromobacterium violaceum CV026 und Agrobacterium tumefaciens NTL4 (pZLR4) (McClean et al. 1997; Luo et al. 2003; Andersen et al. 2001) untersucht.

Als Testmaterial dienten:

Cornus mas-Kernmehl Partikel mit einer Korngröße von ca. 2 mm.

C. mas-Kerntrockenextrakt, der wie in Beispiel 1 beschrieben, hergestellt wurde.

Als Positivkontrolle wurde C30 Furanon in einer Konzentration von 2 mM, das entspricht 506 $\mu$g/ml eingesetzt.

Der QS Inhibierungs-Assay wurde, wie in McClean et al. (1997) beschrieben, durchgeführt, jedoch wurden Modifikationen vorgenommen. A. tumefaciens NTL4 (pZLR4) wurde in Agrobakterium-Medium (für 100 ml: 0,3 g $K_2HPO_4$, 0,1 g $NaH_2PO_4$, 0,1 g $NH_4Cl$, 0,03 g $MgSO_4 \cdot 7H_2O$, 0,015 g KCl, 0,0005 g $CaCl_2$, 0,00025 g $FeSO_4 \cdot 7H_2O$), Chromobacterium violaceum CV026 in Trypton Soja Nährbouillon (TSB-Medium; Merck 1.05459.0500) kultiviert. QS wurde mit einer Endkonzentration von 200 nM N-Hexanoyl-DL-Homoserin Lacton (C6-AHL) aktiviert. Auf den Agar wurde punktuell ein Partikel des Kernmehls und zwei mit je 10 $\mu$l C. mas-Kerntrockenextraktlösung befeuchtetes Filterpapier aufgebracht. Nach entsprechender Inkubation. wie in McClean et al. (1997) beschrieben. wurden die Hemmhöfe um die Testmaterialien sichtbar, von welchen QS unterdrückt vorhanden war.

Die Durchmesser der Hemmhöfe dienten der Quantifizierung der Inhibierung des QS. Die Ergebnisse sind in Tabelle 5 dargestellt.

Für die beiden getesteten C. mas Testmaterialien konnte eine QS inhibierende Wirkung festgestellt werden, wobei der Extrakt, unabhängig von dem verwendeten Biosensor, eine stärkere Wirkung aufwies als das Mehl. Die QS Inhibierung war in den Indikatorstämmen unterschiedlich stark ausgeprägt und zeigte den größten Effekt bei A. tumefaciens NTL4 (pZLR4).

Tabelle 5: QS inhibierende Wirkung von C. mas-Kernmehl und C. mas-Kerntrockenextrakt sowie Furanon auf die Indikatorstämme C. violaceum CV026 und A. tumefaciens NTL4 (pZLR4).

| | Durchmesser Hemmhöfe [mm] | |
|---|---|---|
| | *A. tumefaciens* NTL4 (pZLR4) | *C. violaceum* CV026 |
| C. mas-Kernmehl Partikel | 4 | 0 |
| *C. mas*-Kerntrockenextrakt | | |
| 500 $\mu$g/ml | 10-12 | 4-8 |
| 250 $\mu$g/ml | 7 | 5 |
| 100 $\mu$g/ml | 4 | 3 |
| C30 Furanon 2 mM (=Positivkontrolle) | 12 | 9 |

Beispiel 12: Synergistische antibakterielle Wirkung von KKM und KKTE mit Futtermittelzusatzstoffen.

Die Bestimmung der antibakteriellen Wirkung von Testsubstanzen, welche übliche Futterkomponenten sind, in Kombination mit C. mas-Kernmehl (KKM) und -Kerntrockenextrakt (KKTE), wurde mittels Mikroverdünnungs-Hemmtest nach Richtlinien des "Clinical and Laboratory Standards Institutes" (CLSI Standards M45A, M2 und M7) durchgeführt.

KKM und KKTE wurden wie in Beispiel 1 beschrieben hergestellt. Für den Test in Mikrotiterplattenformat wurden 1000 mg/l KKM bzw. 100 mg/l KKTE eingesetzt, wodurch Hemmwerte höher als 10 % und geringer als 50 % erreicht wurden. Übliche Futtermittelzusatzstoffe, wie Ameisensäure, Kalziumformiat, Benzoesäure, Ascorbinsäure, Vitamin B12 wurden in Kombination mit den genannten Konzentrationen an KKTE getestet, um festzustellen, ob die Hemmwirkung gleich, deutlich höher oder niedriger als mit KKTE alleine liegt. Die Konzentration der Testsubstanzen wurde ebenfalls so gewählt, dass Hemmwerte höher als 10 % und geringer als 50 % erreicht wurden. Falls eine Testsubstanz keine Hemmwirkung zeigte, wurden 1000 mg/l verwendet.

Kalziumformiat, Benzoesäure und Ascorbinsäure zeigten bei 1000 $\mu$g/ml, bzw. Vitamin B12 und Ameisensäure bei 500 $\mu$g/ml jeweils gemeinsam mit KKTE bei 100 $\mu$g/ml eine deutliche synergistische antibakterielle Wirkung gegen Salmonella enteritidis. Die Hemmwirkung der Kombinationen war signifikant höher als jene der Einzelsubstanzen.

Den Synergieindex erhält man durch Division der theoretischen additiven Hemmung durch die tatsächlich gemessene Hemmung durch die Kombination von KKM oder KKTE mit einer Testsubstanz. Ist dieser Index kleiner als 1, handelt es sich um synergistische Wirkung, bei 1 handelt es sich um einen additiven Effekt, während bei einem Index größer als 1 eine antagonistische Wirkung vorliegt.

Unter synergistischer Wirkung wird hier eine Hemmwirkung der Kombination von KKM oder KKTE mit einer Testsubstanz verstanden, die über die theoretische additive Hemmwirkung hinausgeht, unabhängig davon, ob die Testsubstanz alleine eine Hemmwirkung aufweist, oder nicht.

Tabelle 6a: Synergistische antibakterielle Wirkung gegen S. enteritidis von KKM mit verschiedenen Futtermittelzusatzstoffen. Die Hemmwirkung wurde in Prozent angegeben, zusammen mit der Standardabweichung.

| KKM | 0 μg/ml | 1000 μg/ml | Theoretische additive Hemmung | Synergieindex |
|---|---|---|---|---|
| | 0 (± 0) | 30 (± 3) | | |
| + Kalziumformiat 1000 μg/ml | 7 (± 5) | 60 (± 8) | 37 (± 8) | 0,62 |
| + Ameisenäure 500 μg/ml | 31 (± 6) | 75 (± 3) | 61 (± 9) | 0,81 |
| + Benzoesäure 1000 μg/ml | 27 (± 9) | 77 (± 4) | 57 (± 12) | 0,74 |
| + Ascorbinsäure 1000 μg/ml | 5 (± 5) | 53 (± 6) | 35 (± 8) | 0,66 |
| + Vitamin B12 1000 μg/ml | 4 (± 1) | 68 (± 6) | 34 (± 4) | 0,50 |

Tabelle 6b: Synergistische antibakterielle Wirkung gegen S. enteritidis von KKTE mit verschiedenen Futtermittelzusatzstoffen. Die Hemmwirkung wurde in Prozent angegeben, zusammen mit der Standardabweichung.

| KKTE | 0 μg/ml | 100 μg/ml | Theoretische additive Hemmung | Synergieindex |
|---|---|---|---|---|
| | 0 (± 0) | 33 (± 2) | | |
| + Kalziumformiat 1000 μg/ml | 1 (±4) | 63 (± 11) | 34 (± 6) | 0,53 |
| + Ameisenäure 500 μg/ml | 24 (± 10) | 81 (± 6) | 57 (± 12) | 0,70 |
| + Benzoesäure 1000 μg/ml | 34 (± 7) | 78 (± 2) | 67 (± 10) | 0,85 |
| + Ascorbinsäure 1000 μg/ml | 0 (± 5) | 53 (± 4) | 33 (± 4) | 0,62 |
| + Vitamin B12 1000 μg/ml | 0 (± 3) | 68 (± 7) | 33 (± 5) | 0,48 |

Beispiel 13: Akzeptanz von Kornelkirschen-Kernmehl bei Absetzferkel.

Um die Akzeptanz von Kornelkirschen-Kernmehl als Futterzusatz für Ferkel zu prüfen, wurden 9 bzw. 18 kg Kernmehl/t Ferkelaufzuchtfutter in einem ON/OFF Versuch über 4 Wochen eingesetzt.

Als Testmaterial diente ein Kornelkirschen-Kernmehl von *C. mas,* dass, wie in Beispiel 1 beschrieben, hergestellt wurde.

Der Versuchsaufbau enthielt 2 Gruppen, eine Kontrollgruppe und eine Testgruppe, zu je 2 Replikaten in 2 Buchten mit je 9 Aufzuchtferkeln (Alter 35 Tage = Versuchstag 1). Das mittlere Anfangsgewicht der Ferkel-Kontrollgruppe betrug 11,5 kg, das der Ferkel der Testgruppe 11,2 kg. Die Kontrollgruppe erhielt gewöhnliches Ferkelfutter bestehend aus 35 % Weizen (12 % Rohprotein), 14 % Gerste (11 % Rohprotein), 0,2 % Rapsöl, 2,2 % Fasermischung, 3,8 % Mineralfutter, 19,8 % Soja und 25 % Ganzkornsilage (70 % Trockenmasse), die Testgruppe erhielt abwechselnd gewöhnliches Ferkelfutter und mit C. mas-Kernmehl versetztes Futter.

Die Fütterung erfolgt in vier Phasen zu je einer Woche, mit wöchentlicher Umstellung des Futters in der Testgruppe. Es wurde die tägliche Futteraufnahme pro Bucht am Ende jeder Phase bestimmt. Die Entwicklung der Futteraufnahme in den jeweiligen Fütterungsphasen ist in Tabelle 7 dargestellt und zeigt, dass der Zusatz von 9 kg und 18 kg Kernmehl pro Tonne Futter keine Reduktion der Futteraufnahme zur Folge hatte. Alle Ferkel entwickelten sich normal und bei tierärztlichen Kontrollen wurden keine gesundheitlichen Probleme festgestellt.

Tabelle 7: Futteraufnahme von Absetzferkel des Akzeptanzversuches von *C. mas* - Kernmehl.

| Phase | Kontrollgruppe | | Testgruppe | | KKM (kg/t Futter) |
|---|---|---|---|---|---|
| | Futteraufnahme [kg / Tier / Tag] | Standardabweichung | Futteraufnahme [kg / Tier / Tag] | Standardabweichung | |
| 1 | 0,563 | 0,096 | 0,453 | 0,066 | 9 |
| 2 | 0,837 | 0,162 | 0,768 | 0,066 | - |
| 3 | 1,234 | 0,157 | 1,201 | 0,281 | 18 |
| 4 | 1,317 | 0,164 | 1,420 | 0,314 | - |

Beispiel 14: Fütterungsversuch zur Bestimmung der Wirkung von Kornelkirschen-Kernen auf die Leistungsparameter von Aufzuchtferkel.

Die positive Wirkung von Kornelkirschen-Kernen auf Leistungsparameter von Aufzuchtferkel wurde anhand eines Fütterungsversuchs mit *C. mas*-Kernmehl, das, wie in Beispiel 1 beschrieben, hergestellt wurde, gezeigt.

Der Versuchsaufbau enthielt 2 Gruppen, eine Kontrollgruppe und eine Testgruppe, zu je 3 Replikaten in 3 Buchten mit je 10 Aufzuchtferkel. Das mittlere Anfangsgewicht der Kontrollgruppe betrug 8,56 kg $\pm$ 0,88 kg, das der Ferkel der Testgruppe 8,97 kg $\pm$ 1,01 kg. Die Kontrollgruppe erhielt gewöhnliches Ferkelaufzuchtfutter, das Futter der Testgruppe enthielt zusätzlich 1 kg C. mas-Kernmehl/t Futter. Die Kontrollgruppe bekam von Tag 1 bis 14 ein herkömmliches Starterfutter und von Tag 15 bis 42 ein herkömmliches Aufzuchtfutter. Die Testgruppe bekam von Tag 1 bis 14 Starterfutter und von Tag 15 bis 42 Aufzuchtfutter, in welche jeweils 1 kg KKM pro Tonne Futter eingemischt wurden.

Die Tiere wurden an den Tagen 0, 14 und 42 gewogen. Die Futteraufnahme pro Bucht wurde bei Futterwechsel und am Ende des Versuchs bestimmt. Die Testgruppe erreichte bereits nach 14 Tagen ein signifikant höheres Lebendgewicht als die Kontrollgruppe. Nach 42 Tagen betrug der Unterschied 2,78 kg oder knapp 10 % des Gesamtgewichts der Kontrollgruppe. Diese Steigerung spiegelt sich auch in der täglichen Gewichtszunahme und in der erhöhten Futteraufnahme (Tabelle 8) wieder.

Tabelle 8: Effekt von *C. mas*-Kernmehl auf die Leistungsparameter von Aufzuchtferkel.

| Tag | Lebendgew. [kg] | | p-Wert | Tage | Gew.zunahme [g/Tier/Tag] | | p-Wert | Futteraufn. [g/Tier/Tag] | | p-Wert |
|---|---|---|---|---|---|---|---|---|---|---|
| | Kontrollgr. | Testgr. | | | Kontrollgruppe | Testgruppe | | Kontrollgruppe | Testgruppe | |
| 0 | 8,56 | 8,97 | 0,265 | | | | | | | |
| 14 | 11,37 | 12,77 | 0,010 | 1-14 | 201 | 275 | 0,001 | 331 | 415 | 0,110 |
| 42 | 28,72 | 31,50 | 0,010 | 1-42 | 480 | 537 | 0,005 | 823 | 956 | 0,088 |

Beispiel 15: Fütterungsversuch zur Bestimmung der Wirkung von Kornelkirschen-Kernen auf das Durchfallgeschehen von Aufzuchtferkel.

Um die Wirkung von Kornelkirschen-Kernen auf das Durchfallgeschehen von Aufzuchtferkel zu bewerten, wurden zwei unabhängige Ferkel-Fütterungsversuche mit *C. mas*-Kernmehl und *C. officinalis*-Kernmehl durchgeführt.

Es wurden Ferkel am Tag des Absetzens mit einem Alter von zirka 4 Wochen in einen Versuchsstall gebracht und nach einer dreitägigen Anpassungsphase wurde Kornelkirschen-Kernmehl in das Futter eingemischt.

Innerhalb der Versuchsdauer wurde die Durchfallintensität pro Bucht in einem vierstufigen System bewertet: 0 kein Durchfall, 1 leichter Durchfall, 2 mittlerer Durchfall und 3 starker Durchfall. Der durchschnittliche Durchfallscore pro Gruppe entspricht der arithmetisch gemittelten, täglichen Durchfallintensität über alle Buchten der Gruppe. Eine medikamentöse Behandlung mit Enteroxid wurde nur bei Bedarf in der betroffenen Bucht durchgeführt und nur bei einer Durchfallbewertung von größer oder gleich 2, länger als 3 Tage anhaltender leichter Durchfall oder wiederkehrender leichter oder schwerer Durchfall.

Die zur Bewertung des Durchfallgeschehens herangezogenen Parameter waren durchschnittliche Durchfalltage pro Bucht, durchschnittlicher Durchfallscores pro Bucht sowie die durchschnittlich notwendigen medikamentösen Behandlungstage pro Bucht. Die Durchfallbewertung wurde über jenen Zeitraum ermittelt, in welchem es zum Auftreten von Durchfall im Stall kam. Eine eventuell nötige medikamentöse Behandlung erfolgte mit Enteroxid.

Fütterungsversuch A: *Cornus mas*-Kernmehl

Als Testmaterial diente ein Kornelkirschen-Kernmehl von *C. mas,* das, wie in Bsp. 1 beschrieben, hergestellt wurde.

Der Versuchsaufbau enthielt 2 Gruppen, eine Kontrollgruppe und eine Testgruppe, zu je 3 Replikaten in 3 Buchten mit je 10 Aufzuchtferkel. Das mittlere Anfangsgewicht der Ferkel-Kontrollgruppe betrug 8,10 kg $\pm$ 1,34 kg, das der Ferkel der Testgruppe 8,11 kg $\pm$ 1,35 kg. Die Kontrollgruppe erhielt gewöhnliches Ferkelaufzuchtfutter, das Futter der Testgruppe enthielt zusätzlich 1 kg *C. mas*-Kernmehl pro Tonne Futter. Die Futterzusammensetzung und der Fütterungsplan entsprachen jenen von Beispiel 14. Die Versuchsdauer betrug 21 Tage, da ein Durchfallgeschehen nur zwischen den Tagen 6 und 19 beobachtet wurde. Die drei Buchten der Testgruppe zeigten nur für 4,33 Tage leichten Durchfall und eine medikamentöse Behandlung mit Enteroxid war nur in einer Bucht notwendig. Im Gegensatz dazu mussten in der Kontrollgruppe zwei Buchten über sechs Tage hinweg mit Enteroxid behandelt werden, nachdem schwerer Durchfall aufgetreten war. In der Testgruppe wurde signifikant seltener und schwächerer Durchfall im Gegensatz zu der Kontrollgruppe beobachtet. Durch den Zusatz von *C. mas*-Kernmehl wurden pro Bucht die durchschnittlichen Durchfalltage um 28 % von 6,00 auf 4,33, der durchschnittliche Durchfallscore wurde um 53 % von 0,62 auf 0,33 und die durchschnittlichen medikamentösen Behandlungstage wurden um 66 % von 4,00 auf 1,33 Tage reduziert.

Fütterungsversuch B: *Cornus officinalis*-Kernmehl

Als Testmaterial diente ein Kornelkirschen-Kernmehl von *C. officinalis,* das, wie in Bsp. 1 beschrieben, hergestellt wurde.

Der Versuchsaufbau enthielt 2 Gruppen, eine Kontrollgruppe und eine Testgruppe, zu je 3 Replikaten in 3 Buchten mit je 10 Aufzuchtferkel. Das mittlere Anfangsgewicht der Ferkel-Kontrollgruppe betrug 8,49 kg $\pm$ 1,56 kg, das der Ferkel der Testgruppe 8,48 kg $\pm$ 1,56. Die Futterzusammensetzung und der Fütterungsplan entsprachen jenen von Beispiel 14. Die Versuchsdauer betrug 21 Tage, da ein Durchfallgeschehen nur zwischen den Tagen 6 und 14 beobachtet wurde. Durchfall trat in der Testgruppe viel seltener auf als in der Kontrollgruppe und es waren in der Testgruppe nur durchschnittlich 2 Behandlungstage mit Enteroxid nötig, verglichen mit 8 in der Kontrollgruppe. Durch den Zusatz von C. *officinalis*-Kernmehl wurden pro Bucht die durchschnittlichen Durchfalltage um 61 % von 7,66 auf 3,00, der durchschnittliche Durchfallscore wurde um 36 % von 0,59 auf 0,38 und die durchschnittlichen medikamentösen Behandlungstage wurden um 75 % von 8,00 auf 2,00 Tage reduziert.

Beispiel 16: Fütterungsversuch von Aufzuchtferkel mit Kornelkirschen-Kernmehl und Kornelkirschen-Kernextrakt.

Zum Vergleich der Wirkung von KKM und KKTE wurden diese aus demselben Ausgangsmaterial hergestellt und ein Fütterungsversuch mit Ferkeln über 14 Tage durchgeführt.

Als Testmaterial diente KKM von *C. mas,* dass durch Zerkleinerung von Kernen mit einer Schneidmühle mit einem Siebeinsatz von 1,0 mm hergestellt wurde. Der KKTE wurde durch Extraktion von 20 kg der gemahlenen Kornelkirschen-Kerne mittels Rührbottich mit 100 l, 50 % Ethanol über 24 h bei 25 °C gewonnen. Das Extraktionsmittel wurde anschließend per Rotations-Evaporator bei 60 °C entfernt und der Extrakt zur Trockene gebracht. Aus der eingesetzten Menge Rohstoff konnten 1,63 kg Trockenextrakt gewonnen werden (Droge-Extrakt-Verhältnis DEV= ca. 12:1).

Für den Fütterungsversuch wurden die Ferkel in 3 Gruppen zu je 3 Buchten mit 10 Ferkel aufgeteilt. Gruppe 1 bekam ein herkömmliches Ferkelfutter ohne Zusatzstoffe, Gruppe 2 bekam zusätzlich KKM (Einmischrate 1000 g/t Fertigfutter) und Gruppe 3 bekam zusätzlich KKTE (Einmischrate von 83 g/t Fertigfutter). Dabei wurde die Einmischrate des KKTE von Gruppe 3 so gewählt, dass sie derjenigen an KKM von Gruppe 2 entspricht (bei einem DEV von 12:1).

Alle Ferkel wurden prophylaktisch mit Enteroxid behandelt und es kam zu keinem Auftreten von Durchfall, deshalb ist in diesem Versuch keine Aussage über Durchfallwirkung der getesteten Produkte möglich.

Gruppe 2 (KKM) und Gruppe 3 (KKTE) zeigen eine höhere tägliche Zuwachsrate, eine verbesserte Futteraufnahme sowie eine deutlich verbesserte Futterverwertungsrate gegenüber der Kontrollgruppe (Tabelle 9).

Tabelle 9: Ergebnisse eines Fütterungsversuchs mit KKM (Gruppe 2), KKTE (Gruppe 3) und ohne Zusatzstoffe (Kontrolle, Gruppe 1).

| Parameter | Gruppe | Mittelwert |
|---|---|---|
| Tägliche Gewichtszunahme (TZ) [g /Ferkel /Tag] | 1 | 254 |
| | 2 | 276 |
| | 3 | 280 |
| Futteraufnahme (FA) [g/ Ferkel /Tag] | 1 | 430 |
| | 2 | 439 |
| | 3 | 445 |
| Futterverwertungsrate [FA / TZ] | 1 | 1,70 |
| | 2 | 1,60 |
| | 3 | 1,59 |

Beispiel 17: Feldversuche mit Absetzferkeln

Um die Wirkung von Kornelkirschen-Kernen auf die Leistung bei Aufzuchtferkeln im Routinebetrieb zu bewerten, wurde ein Feldversuch mit 240 Absetzferkeln durchgeführt.

Als Testmaterial diente ein Kornelkirschen-Kernmehl von C. mas, welches wie in Bsp. 1 beschrieben, hergestellt wurde.

Die Tiere wurden in 4 Gruppen zu je 60 Ferkeln eingeteilt, wobei zwei Gruppen ein geringeres Startgewicht und zwei Gruppen ein höheres Startgewicht hatten. Je einer Gruppe mit geringerem und höherem Stargewicht wurde C. mas-Kernmehl als Zusatzstoff ins Futter gemischt. Die Einmischrate lag bei 3,0 kg/t herkömmliches Starterfutter. Die zwei verbleibenden Gruppen dienten als Kontrollgruppen. Alle Gruppen erhielten prophylaktisch Enteroxid. Von allen Gruppen wurde jeweils das gemittelte Gesamtgewicht zu Beginn und am Ende des Versuchs, nach 14 Tagen, gemessen (Tabelle 10). Die Wirkung des C. mas-Kernmehls auf das Körpergewicht war deutlich. Die Steigerung betrug 68 % bei den Ferkeln mit geringerem Startgewicht um 16 % bei den Ferkeln mit größerem Startgewicht, bezogen jeweils auf die Kontrollgruppen.

Tabelle 10: Wirkung von *C. mas*-Kernmehl (3 kg/t Fertigfutter) auf das Lebendgewicht von Absetzferkel im Feldversuch.

| Lebendgewicht pro Ferkel [kg] | Kontrollgruppe leicht | Versuchsgruppe leicht | Kontrollgruppe schwer | Versuchsgruppe schwer |
|---|---|---|---|---|
| Tag 0 | 7,97 | 7,90 | 8,60 | 8,63 |
| Tag 14 | 9,20 | 9,97 | 12,53 | 13,20 |

Beispiel 18: Fütterungsversuch mit Masthühnern

Um den Effekt von Kornelkirschen-Kernen auf die Leistungsparameter von Geflügel zu bewerten, wurde ein Hühner-Fütterungsversuch mit *C. mas*-Kernmehl durchgeführt. Dazu wurden 420 Ross Küken in drei Versuchsgruppen zu je 7 Buchten mit jeweils 20 Küken aufgeteilt. Die Kontrollgruppe erhielt gewöhnliches Hühnerfutter, die zwei Versuchsgruppen erhielten zusätzlich *C. mas*-Kernmehl als Futterzusatzstoff in einer Einmischrate von 1,0 kg (Testgruppe 1) und 0,5 kg/t (Testgruppe 2) Futtermittel. Die Hühner wurden über einen Zeitraum von 35 Tagen aufgezogen und am Tag 0, 14 und 35 gewogen.

Die Leistungsparameter Lebendgewicht, Futteraufnahme sowie Futterverwertungsrate sind in Tabelle 12 und 13 dargestellt. Die Fütterung von *C.mas*-Kernmehl steigerte in beiden Einmischraten das Lebendgewicht der Masthühner im Vergleich zur Kontrollgruppe deutlich, wobei kein signifikanter Unterschied zwischen den Testgruppen bestand. Die Futteraufnahmen in den Testgruppen waren geringer als in der Kontrollgruppe. Das gestiegene Lebendgewicht bei geringerem Futtermitteleinsatz führt zu einer deutlichen Verbesserung der Futterverwertungsrate der Testgruppen im Vergleich zur Kontrollgruppe. Die Futterverwertungsrate betrug nach 35 Tagen bei der Kontrollgruppe 1,86 kg/kg (kg Futtermittel/kg Lebendgewicht), bei Testgruppe 1 1,74 kg/kg und bei Testgruppe 2 1,79 kg/kg.

Tabelle 11: Wirkung von *C. mas*-Kernmehl auf das durchschnittliche Lebendgewicht von Masthühnern.

| Tag | Kontrollgruppe [g] | Testgruppe 1 [g] | Testgruppe 2 [g] |
| --- | --- | --- | --- |
| 0 | 37 ± 0,80 | 37 ± 0,75 | 37 ± 0,92 |
| 14 | 321 ± 24,25 | 328 ± 24,65 | 334 ± 12,28 |
| 35 | 1798 ± 302,62 | 1871 ± 320,30 | 1864 ± 268,11 |

Tabelle 12: Wirkung von *C. mas*-Kernmehl auf die durchschnittliche Futteraufnahme sowie die Futterverwertungsrate von Masthühnern in einem Beobachtungszeitraum von 35 Tagen.

| Gruppe | Futteraufnahme [g] | Futterverwertungsrate [kg / kg] |
| --- | --- | --- |
| Kontrollgruppe | 3270 ± 231,50 | 1,86 ± 0,15 |
| Testgruppe 1 | 3193 ± 280,69 | 1,74 ± 0,09 |
| Testgruppe 2 | 3255 ± 117,70 | 1,79 ± 0,11 |

Beispiel 19: Charakterisierung des Kornelkirschen-Kerntrokenextrakts durch Identifizierung und Quantifizierung ausgewählter Inhaltsstoffe mittels Massenspektrometrie

Ethanolische Kornelkirschen-Kerntrockenextrakte aus Kernen von *C. mas* und *C. officinalis* wurden, wie in Beispiel 1 beschrieben, hergestellt. Extraktionsmittel: Verhältnis 1:5 (v/v) Kernmehl:Ethanol-Wassermischung mit 70 Vol.-% Ethanol, Extraktionsdauer: 24 h.

Wässrige Kornelkirschen-Kerntrockenextrakte aus Kernen von *C. mas* und *C. officinalis* wurden, wie in Beispiel 1 beschrieben, hergestellt. Extraktionsmittel: Verhältnis 1:5 (v/v) Kernmehl:reinem Wasser, Extraktionsdauer: 24 h.

Die Messungen wurden nach der in METLIN (Smith, O'Maille et al. 2005; Zhu, Schultz et al. 2013) beschriebenen Methode mittels Flüssigchromatographie gekoppelt an ein Elektrospray-Flugzeit-Massenspektrometer (LC-ESI-TOF-MS) durchgeführt, um die in der METLIN-Datenbank (MDB, http://metlin.scripps.edu/index.php) hinterlegten Retentionszeiten der Substanzen mit den eigenen Messungen vergleichen zu können.

Die Identifizierung der Substanzen erfolgte mittels zweier unabhängiger Kriterien: der akkuraten Masse (AM) und der Retentionszeit. Aus der akkuraten Masse (in Da/mol, auf 4 Dezimalstellen genau, Massengenauigkeit +/-2 ppm) wurde eine Summenformel ermittelt (Mass Profiler Professional Software, Agilent). Diese Summenformel wurde mit der METLIN Datenbank abgeglichen, in welcher zu den entsprechenden Summenformeln auch Strukturformeln und damit Substanznamen hinterlegt sind. Stimmte auch die Retentionszeit mit jener in der Datenbank überein, so galt die Substanz als identifiziert. Für die Substanzen Gallussäure, Ellagsäure und Pentagalloylglucose wurden zusätzlich die genauen Retentionszeiten mittels Injektion von authentischen Standards (AS) ermittelt.

Die Inhaltsstoffe in den Extrakten bzw. den Kernen (exhaustive Extraktion angenommen) wurden über die Peakflächen quantifiziert. Als Referenz dienten zum einen Referenzsubstanzen von Gallussäure (1 mg/mL, Sigma G7384), Ellagsäure (95 %, 0,1 mg/mL, Sigma 2250) und Pentagalloylglucose (1 mg/mL, Sigma G7548) und zum anderen die Gesamtpeakfläche des Chromatrogramms für Loganin und Dihydroquercentin.

Identifikation und Quantifizierung der ausgewählten Inhaltsstoffe ist exemplarisch für C. mas in Tabelle 13 und 14 für einen ethanolischen und wässrigen Extrakt angegeben. Die Analyse von C. officinalis KKTE ergab jedoch nahezu idente Messwerte, sodass die Zusammensetzung beider KKTE als gleichwertig angesehen werden kann.

Tabelle 13: Inhaltsstoffe eines C. mas KKTE, hergestellt durch Extraktion mit 70% Ethanol. Das Droge-Extrakt-Verhältnis (DEV) betrug 12:1. Die Messungen wurden in 3-facher Wiederholung durchgeführt.

| KKTE Inhaltsstoff | AM (Da/mol) | Gehalt (mg pro kg KKTE) | Gehalt (mg pro kg Kernmehl) | Identifizierung |
|---|---|---|---|---|
| Gallussäure | 170,1195 | 769,2 | 64,1 | AS |
| Ellagsäure | 302,1926 | 10.384,8 | 865,4 | AS |
| Pentagalloylglucose | 940,6772 | 5.088 | 424,0 | AS |
| Loganin* | 390,3823 | 519,9 | 43,3 | MDB |
| Dihydroquercetin* | 304,2516 | 172,8 | 14,4 | MDB |
| *Quantifizierung des Gehalts erfolgte unter der Annahme dass sämtliche Substanzen des Kernextrakts zur Gesamtpeakfläche beitragen. | | | | |

Bei der Extraktion mit 100% Ethanol wurden überraschenderweise im Wesentlichen dieselben Inhaltsstoffe identifiziert und auch deren Gehalte im Extrakt war mit dem 70%igem ethanolischen Extrakt nahezu ident.

Tabelle 14: Inhaltsstoffe eines C. mas KKTE, hergestellt durch Extraktion mit 100% Wasser. Das Droge-Extrakt-Verhältnis (DEV) betrug 9,8:1. Die Messungen wurden in 3-facher Wiederholung durchgeführt.

| KKTE Inhaltsstoff | AM (Da/mol) | Gehalt (mg pro kg KKTE) | Identifizierung |
|---|---|---|---|
| Gallussäure | 170,1195 | 690,1 | AS |
| Ellagsäure | 302,1926 | 5736,4 | AS |
| Pentagalloylglucose | 940,6772 | Nd | AS |
| Loganin* | 390,3823 | 532,4 | MDB |
| Dihydroquercetin* | 304,2516 | 10,5 | MDB |
| Nd ... nicht detektierbar<br>*Quantifizierung des Gehalts erfolgte unter der Annahme dass sämtliche Substanzen des Kernextrakts zur Gesamtpeakfläche beitragen. | | | |

Beispiel 20: Vergleich der antibakteriellen Wirkung von reinen Inhaltsstoffen im Vergleich zum KKTE

Wie in den Beispielen 3 und 4 beschrieben liegt die MHK50 eines C. mas Kornelkirschen-Kerntrockenextrakts aus 70 Vol% Ethanol auf den enteropathogenen Bakterienstamm E. coli O128:H2 zwischen 78 und 156 mg/l, die MHK 50 des Extrakts aus reinem Wasser liegt zwischen 156 und 313 mg/l. Untersuchungen der reinen Substanzen Gallussäure, Ellagsäure, Pentagalloylglucose und Loganin in demselben Versuchsansatz zeigten jeweils eine MHK50 > 500 mg/l.

Aus diesen Ergebnissen geht klar hervor, dass keiner der identifizierten Inhaltsstoffe für sich alleine für die außergewöhnlich guten antibakteriellen Eigenschaften von KKTE verantwortlich ist.

Überraschenderweise zeigen diese Ergebnisse klar, dass der in den Kernen bekannte Inhaltsstoff Pentagalloylglucose nicht wesentlich zur antibakteriellen Wirkung beiträgt, da die ethanolischen und die wässrigen Extrakte annähernd die gleichen minimalen Hemmkonzentrationen für E. coli O128:H2, S. typhimurium und C. perfringens Toxin Typ A (siehe Beispiel 4) aufweisen, die Substanz Pentagalloylglucose jedoch aufgrund der Unlöslichkeit in Wasser nur in den ethanolischen Extrakten vorkommt.

Beispiel 21: Chromatographischer Vergleich von KKTE von C. mas und C. officinalis verschiedener Herkünfte

Die fünf Kornelkirschen-Kerntrockenextrakte aus Beispiel 3 wurden mittels Hochdruck Flüssigkeitschromatographie (HPLC), die an einen Diodenarray Detektor (DAD) gekoppelt war, analysiert. Dies ermöglicht die Darstellung aller im DAD sichtbaren Substanzen in einem Chromatogramm. Ein Vergleich der HPLC-DAD Chromatogramme gibt Auskunft wie ähnlich die Zusammensetzung einzelner Proben ist.

Als HPLC wurde folgendes Equipment verwendet: Degasser G1379B Autosampler G1329B, Binary pump G1312A (1100 series), Thermostated Column Compartment G1316A, Standard Flow cell G1315D 018. Als Detektor diente ein Diode Array Detektor G1315D (1200 series). Als Säule wurde eine Agilent Zorbax Sb-Aq 250x4.6 mm verwendet. Lösungsmittel A war Wasser mit 0,1% Ameisensäure und Lösungsmittel B war Acetonitril mit 0,1% Ameisensäure. Es wurde folgender Grandient zur Trennung der Substanzen verwendet: 0,00 min (10% B); 47.00 min (22 % B); 50,00 min (25% B), 51,00 min (100% B); 55,00 min (100% B); 56,00 min (10% B); 60,00 min (10% B). Das Injektionsvolumen war 20 μl, die Flussrate 1 ml/min und die Temperatur 25°C. Der DAD zeichnete folgende einzelne Wellenlängen auf: 220 nm, 254 nm, 280 nm, 320 nm, 350 nm, 580 nm, sowie das gesamte Spektrum von 190 nm bis 900 nm.

Eine Übersicht der fünf HPLC-DAD Chromatogramme ist in Figur 1 gezeigten. Darin ist deutlich sichtbar, dass es keine wesentlichen Unterschiede in der Zusammensetzung der Kerne verschiedener Herkünfte (Österreich, Deutschland, Türkei) und Arten (*C. mas* und *C. officinalis*) gibt. Die chemischen Zusammensetzungen der Kornelkirschen-Kernextrakte von Kernen verschiedener Herkunft und verschiedener Arten unterscheiden sich somit nicht wesentlich voneinander.

Beispiel 22: Charakterisierung der Zusammensetzung von Kornelkirschen-Kernmehl

Die fünf unterschiedlichen Kornelkirschen-Kernmehle aus Beispiel 3 und 21 von verschiedener Herkunft und Art wurden mittels eine erweiterte Weender Futtermittel- und Spurenelementanalyse nach VDLUFA (Die chemische Untersuchung von Futtermitteln. Methodenbuch. Band III. VDLUFA-Verlag, 2007) näher charakterisiert. Die Mittelwerte, sowie maximale und minimale Werte der jeweiligen Messparameter sind in Tabelle 15 zusammengefasst. Die Zusammensetzungen der unterschiedlichen Kornelkirschen-Kernmehle sind sehr ähnlich und können daher als gleichwertig angesehen werden.

Tabelle 15: Charakterisierung von KKM durch erweiterte Weender Analyse. Fünf Proben verschiedener Herkunft wurden analysiert. Alle Werte beziehen sich auf 100% Trockenmasse (TM), außer dem Wert für TM selbst, welcher sich auf die Frischmasse bezieht.

|  | Mittelwert [g pro kg] | Maximum [g pro kg] | Minimum [g pro kg] |
|---|---|---|---|
| Trockenmasse | 918 | 877 | 942 |
| Rohasche | 14.7 | 11.0 | 16.0 |
| Rohprotein | 41.2 | 36.0 | 48.0 |
| Rohfett | 57.3 | 49.0 | 67.0 |
| Stärke | 10.0 | 11,3 | 8,9 |
| Zucker | 11.0 | 11,9 | 10.1 |
| Rohfaser | 556 | 485 | 598 |
| Stickstofffreie Extraktstoffe | 330 | 293 | 361 |
| Neutrasdetergenzfaser | 711 | 646 | 752 |
| Säuredetergenzfaser | 524 | 457 | 558 |
| Säuredetergenzlignin | 232 | 198 | 250 |
| Ca | 4.1 | 3.8 | 4.2 |
| P | 1.1 | 0.9 | 1.4 |
| Mg | 0.6 | 0.5 | 0.9 |
| K | 1.4 | 1.0 | 2.3 |
| Na | 0.2 | 0.1 | 0.3 |
| Fe | 37.3 | 34.4 | 40.9 |
| Mn | 5.2 | 4.3 | 6.9 |
| Zn | 19.5 | 15.1 | 25.1 |
| Cu | 7.0 | 5.3 | 8.8 |

**Patentansprüche**

1. Zusatzstoff für Futtermittel, Nahrungsmittel, Trinkwasser oder pharmazeutischen Präparate hergestellt aus *wenigstens einer Kornelkirschen Spezies, insbesondere Cornus mas, Cornus officinalis, Cornus chinensis oder Cornus eydeana*, **dadurch gekennzeichnet, dass** wenigstens eine Komponente gewählt aus Kornelkirschen-Kernmehl und/oder Kornelkirschen-Kernextrakt sowie zusätzlich wenigstens eine weitere aus chemischer Synthese und/oder mikrobieller Fermentation erhaltene Substanz, gewählt aus Kalziumformiat, Benzoesäure, Ascorbinsäure, Ameisensäure oder Vitamin B12 und gegebenenfalls wenigstens ein Bestandteil gewählt aus Trägermaterialien, Formulierungshilfsstoffen und/oder biologisch wirksamen Komponenten enthalten sind.

**2.** Zusatzstoff nach Anspruch 1, **dadurch gekennzeichnet, dass** die eingesetzte Kornelkirschenart wenigstens eine aus *Cornus mas* und *Cornus officinalis* ist.

**3.** Zusatzstoff nach einem der Ansprüche 1 oder 2, **dadurch gekennzeichnet, dass** das Kornelkirschen-Kernmehl Korngrößen von 50 $\mu$m bis 5 mm, bevorzugt von 100 $\mu$m bis 2 mm aufweist.

**4.** Zusatzstoff nach Anspruche 1, 2 oder 3, **dadurch gekennzeichnet, dass** das Kornelkirschen-Kernmehl im Futtermittel in einer Konzentration von bis zu 20 kg/t, bevorzugt in einem Bereich von 0,1 kg/t bis 10 kg/t, insbesondere bevorzugt in einem Bereich von 0,5 kg/t bis 5 kg/t enthalten ist.

**5.** Zusatzstoff nach einem der Ansprüche 1 bis 4, **dadurch gekennzeichnet, dass** der Kornelkirschen-Kernextrakt ein wässriger, ein organischer, ein wässrig/organischer Kornelkirschen-Kernextrakt oder ein daraus hergestellter Kornelkirschen-Kerntrockenextrakt ist.

**6.** Zusatzstoff nach Anspruch 5, **dadurch gekennzeichnet, dass** der Kornelkirschen-Kernextrakt pro Kilogramm zwischen 70 mg und 7000 mg, bevorzugt zwischen 350 mg und 1500 mg Gallussäure und/oder zwischen 600 mg und 100000 mg, bevorzugt zwischen 2500 mg und 20000 mg Ellagsäure und/oder zwischen 50 mg und 5000 mg, bevorzugt zwischen 250 mg und 1000 mg Loganin und/oder zwischen 1 mg und 2000 mg, bevorzugt zwischen 5 mg und 350 mg Dihydroquercentin enthält.

**7.** Zusatzstoff nach Anspruch 5 oder 6, **dadurch gekennzeichnet, dass** der Kornelkirschen-Kernextrakt im Futtermittel in einer Konzentration von bis zu 10 kg/t, bevorzugt in einem Bereich von 1 g/t bis 5 kg/t, insbesondere bevorzugt in einem Bereich von 5 g/t bis 1 kg/t enthalten ist.

**8.** Zusatzstoff nach einem der Ansprüche 1 bis 7, **dadurch gekennzeichnet, dass** wenigstens ein weiterer Bestandteil, wie ein Formulierungshilfsstoff, wie ein weiterer Pflanzenbestandteil aus wenigstens einer Kornelkirschen Spezies, gewählt aus der Gruppe Fruchtfleisch, Blätter, Rinde, Stamm, Blüten und Wurzeln und/oder als Trägermaterial, ein physiologisch verträgliches Trägermaterial, vorzugsweise Maltodextrin, Cyclodextrin, Kalziumcarbonat, Stärke, Dinatriumsulfat, Talkum, Saccharose, Bentonite und/oder Zeolithe; eine Futterkomponente wie Getreide; Nebenprodukte aus der Getreideverarbeitung wie Trockenschlempe; Mais, Weizen, Weizenkleie, Reis, Reiskleie, Silage, Öle oder Fette aus pflanzlichen oder tierischen Quellen, Mineralien und/oder Vitamine und/oder eine biologisch wirksame Komponente, gewählt aus der Gruppe Probiotika, Prebiotika, Futtermittelenzyme, Hefen, Hefebestandteile, Säuren, vorzugsweise organische Säuren oder Salze davon; Phytogene; immunstimulierende oder bioaktive Pflanzeninhaltsstoffe und Mykotoxin-deaktivierende Substanzen enthalten ist.

**9.** Zusatzstoff nach einem der Ansprüche 1 bis 8, **dadurch gekennzeichnet, dass** wenigstens ein weiteres Mittel zur Prophylaxe und/oder Behandlung von Durchfallerkrankungen und/oder Verdauungsstörungen enthalten ist.

**10.** Zusatzstoff nach Anspruch 9, **dadurch gekennzeichnet, dass** das weitere Mittel zur Prophylaxe und/oder Behandlung von Durchfallerkrankungen ausgewählt aus der Gruppe Antibiotika, Kokzidiostatika, Enteroxid und Zinkoxid ist.

**11.** Kornelkirschen-Kernmehl und/oder Kornelkirschen-Kernextrakt aus wenigstens einer Kornelkirschen Spezies, insbesondere *Cornus mas, Cornus officinalis, Cornus chinensis* oder *Cornus eydeana* sowie zusätzlich wenigstens eine weitere aus chemischer Synthese und/oder mikrobieller Fermentation erhaltene Substanz, gewählt aus Kalziumformiat, Benzoesäure, Ascorbinsäure, Ameisensäure oder Vitamin B12 sowie gegebenenfalls wenigstens ein Bestandteil gewählt aus Trägermaterialien, Formulierungshilfsstoffen und/oder biologisch wirksamen Komponenten zur Verwendung zur Prophylaxe und/oder Behandlung von Durchfallerkrankungen, insbesondere in der Nutztierhaltung.

**Claims**

**1.** An additive for feeds, foods, drinking water or pharmaceutical preparations, prepared from at least one cornelian cherry species, in particular *Cornus mas, Cornus officinalis, Cornus chinensis* or *Cornus eydeana,* **characterized**

**in that** at least one component selected from cornelian cherry stone powder and/or cornelian cherry stone extract as well as, in addition, at least one further substance obtained from chemical synthesis and/or microbial fermentation, selected from calcium formate, benzoic acid, ascorbic acid, formic acid or vitamin B12, and optionally at least one component selected from carrier substrates, formulation excipients and/or biologically active components, are contained.

2. An additive according to claim 1, **characterized in that** the used cornelian cherry species is at least one from *Cornus mas* and *Cornus officinalis.*

3. An additive according to claim 1 or 2, **characterized in that** the cornelian cherry stone powder comprises particle sizes ranging from 50 μm to 5 mm, preferably from 100 μm to 2 mm.

4. An additive according to claim 1, 2 or 3, **characterized in that** the cornelian cherry stone powder is contained in the feed at a concentration of up to 20 kg/t, preferably ranging from 0.1 kg/t to 10 kg/t, particularly preferably ranging from 0.5 kg/t to 5 kg/t.

5. An additive according to any one of claims 1 to 4, **characterized in that** the cornelian cherry stone extract is an aqueous, an organic, an aqueous/organic cornelian cherry stone extract, or a cornelian cherry stone dry extract prepared therefrom.

6. An additive according to claim 5, **characterized in that** the cornelian cherry stone extract per kilogram comprises between 70 mg and 7000 mg, preferably between 350 mg and 1500 mg, gallic acid and/or between 600 mg and 100000 mg, preferably between 2500 mg and 20000 mg, ellagic acid and/or between 50 mg and 5000 mg, preferably between 250 mg and 1000 mg, loganin and/or between 1 mg and 2000 mg, preferably between 5 mg and 350 mg, dihydroquercetin.

7. An additive according to claim 5 or 6, **characterized in that** the cornelian cherry stone extract is contained in the feed at a concentration of up to 10 kg/t, preferably ranging from 1 g/t to 5 kg/t, particularly preferably ranging from 5 g/t to 1 kg/t.

8. An additive according to any one of claims 1 to 7, **characterized in that** it comprises at least one further component such as a formulation excipient, such as a further plant component from at least one cornelian cherry species, selected from the group of fruit pulp, leaves, bark, stem, flowers and roots, and/or as carrier material a physiologically compatible carrier material, preferably maltodextrin, cyclodextrin, calcium carbonate, starch, disodium sulfate, talcum powder, sucrose, bentonite and/or zeolithe; a feed component such as a cereal, by-products from cereal processing such as distillers dried grains with solubles, corn, wheat, wheat bran, rice, rice bran, silage, oils or fats from plant or animal sources; minerals and/or vitamins; and/or a biologically active component selected from the group of probiotics, prebiotics, feed enzymes, yeasts, yeast components, acids, preferably organic acids, or salts thereof; phytogens; immunostimulating or bioactive plant ingredients; and mycotoxin-deactivating substances.

9. An additive according to any one of claims 1 to 8, **characterized in that** it comprises at least one further agent for the prophylaxis and/or treatment of diarrhoeal diseases and/or indigestions.

10. An additive according to claim 9, **characterized in that** the further agent for the prophylaxis and/or treatment of diarrhoeal diseases is selected from the group of antibiotics, coccidiostats, Enteroxid and zinc oxide.

11. A cornelian cherry stone powder and/or a cornelian cherry stone extract from at least one cornelian cherry species, in particular *Cornus mas, Cornus officinalis, Cornus chinensis* or *Cornus eydeana* as well as, in addition, at least one further substance obtained from chemical synthesis and/or microbial fermentation, selected from calcium formate, benzoic acid, ascorbic acid, formic acid or vitamin B12, and optionally at least one component selected from carrier substrates, formulation excipients and/or biologically active components, for use for the prophylaxis and/or treatment of diarrhoeal diseases, in particular in livestock farming.

**Revendications**

1. Additif pour des aliments pour animaux, des denrées alimentaires, de l'eau potable ou des préparations pharmaceutiques fabriquées à partir d'au moins une espèce de cornouiller, en particulier le *Cornus mas,* le *Cornus officinalis,* le *Cornus chinensis* ou le *Cornus eydeana*, **caractérisé en ce que** sont contenus au moins un composant choisi parmi de la farine de graine de cornouiller et/ou de l'extrait de graines de cornouiller, ainsi qu'en supplément au moins une autre substance obtenue d'une synthèse chimique et/ou d'une fermentation microbienne, choisie parmi du formiate de calcium, de l'acide benzoïque, de l'acide ascorbique, de l'acide formique ou de la vitamine B12 et éventuellement au moins un constituant choisi parmi des matériaux de support, des adjuvants de formulation et/ou des composants ayant une action biologique.

2. Additif selon la revendication 1, **caractérisé en ce que** le type de cornouiller employé est au moins un cornouiller parmi le *Cornus mas* et le *Cornus officinalis.*

3. Additif selon l'une des revendications 1 ou 2, **caractérisé en ce que** la farine de graines de cornouiller présente des granulométries allant de 50 μm à 5 mm, de manière préférée allant de 100 μm à 2 mm.

4. Additif selon la revendication 1, 2 ou 3, **caractérisé en ce que** la farine de graines de cornouiller dans l'aliment pour animaux est contenue en une concentration allant jusqu'à 20 kg/t, de manière préférée dans une plage allant de 0,1 kg/t à 10 kg/t, en particulier de manière préférée dans une plage allant de 0,5 kg/t à 5 kg/t.

5. Additif selon l'une des revendications 1 à 4, **caractérisé en ce que** l'extrait de graines de cornouiller est un extrait de graines de cornouiller aqueux, organique, aqueux/organique ou un extrait sec de graines de cornouiller fabriqué sur cette base.

6. Additif selon la revendication 5, **caractérisé en ce que** l'extrait de graines de cornouiller contient par kilogramme entre 70 mg et 7000 mg, de manière préférée entre 350 mg et 1500 mg d'acide gallique et/ou entre 600 mg et 100 000 mg, de manière préférée entre 2500 mg et 20 000 mg d'acide ellagique et/ou entre 50 mg et 5000 mg, de manière préférée entre 250 mg et 1000 mg de loganine et/ou entre 1 mg et 2000 mg, de manière préférée entre 5 mg et 350 mg de dihydroquercétine.

7. Additif selon la revendication 5 ou 6, **caractérisé en ce que** l'extrait de graines de cornouiller dans l'aliment pour animaux est contenu en une concentration allant jusqu'à 10 kg/t, de manière préférée dans une plage allant de 1 g/t à 5 kg/t, en particulier de manière préférée dans une plage allant de 5 g/t à 1 kg/t.

8. Additif selon l'une des revendications 1 à 7, **caractérisé en ce qu'**au moins un autre constituant, tel qu'un adjuvant de formulation, tel qu'un autre constituant végétal issu d'au moins une espèce de cornouiller, choisi parmi le groupe de la pulpe, des feuilles, de l'écorce, du tronc, des fleurs et des racines et/ou en tant que matériau de support, un matériau de support physiologiquement compatible, de préférence de la maltodextrine, de la cyclodextrine, du carbonate de calcium, de l'amidon, du sulfate de disodium, du talc, du saccharose, de la bentonite et/ou de la zéolithe ; un composant d'aliment pour animaux tels que des céréales ; des produits secondaires issus de la transformation de céréales tels que la drêche sèche ; du maïs, du blé, du son de blé, du riz, du son de riz, de l'ensilage, des huiles ou matières grasses d'origine végétale ou animale, des minéraux et/ou des vitamines et/ou un composant biologiquement actif, choisi parmi le groupe des probiotiques, des prébiotiques, des enzymes d'aliment pour animaux, des levures, des constituants de levure, des acides, de préférence des acides organiques ou des sels de ceux-ci ; des phytogènes ; des phytonutriments immunostimulants ou bioactifs et des substances de désactivation de mycotoxine.

9. Additif selon l'une des revendications 1 à 8, **caractérisé en ce qu'**au moins un autre produit de prophylaxie et/ou de traitement de la diarrhée et/ou de troubles digestifs est contenu.

10. Additif selon la revendication 9, **caractérisé en ce que** l'autre produit de prophylaxie et/ou de traitement de diarrhées est choisi parmi le groupe des antibiotiques, des coccidiostatiques, de l'entéroxyde et de l'oxyde de zinc.

11. Farine de graines de cornouiller et/ou extrait de graines de cornouiller issus d'au moins une espèce de cornouiller, en particulier le *Cornus mas,* le *Cornus officinalis,* le *Cornus chinensis* ou le *Cornus eydeana,* ainsi qu'au moins une autre substance obtenue d'une synthèse chimique et/ou d'une fermentation microbienne, choisie parmi du formiate de calcium, de l'acide benzoïque, de l'acide ascorbique, de l'acide formique ou de la vitamine B12 et éventuellement au moins un constituant choisi parmi des matériaux de support, des adjuvants de formulation et/ou des composants ayant une action biologique pour être utilisés aux fins de la prophylaxie et/ou du traitement de diarrhées, en particulier dans l'élevage d'animaux de rente.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- KR 2008098128 A

- DE 19938931 A1

**In der Beschreibung aufgeführte Nicht-Patentliteratur**

- **LEE et al.** *Biological & pharmaceutical bulletin,* 2011, vol. 34 (3), 443-6
- **LI YAN et al.** *A kind of core of Fructus Corni extract, and its application in the preparation of composition with blood presure lowering effect*
- Database. 201210000983-A
- **N. MENKOVIC et al.** *Journal of Ethnopharmacology,* 2011, vol. 133, 97-107
- **VAREED et al.** *Life sciences,* 2006, vol. 78 (7), 777-84
- **QIU-YUN (JENNY) XIANG.** *Evolution,* 2005, vol. 59 (8), 1685-1700
- **MATTIG et al.** *Appl, Parasität,* 1993, vol. 34, 139-142
- **SCHUBERT et al.** *Parasität Res.,* 2005, vol. 197, 59-62
- **GANNER et al.** *Micobiology Methods,* 2010, vol. 83 (2), 168 ff
- Die chemische Untersuchung von Futtermitteln. Methodenbuch. VDLUFA-Verlag, 2007, vol. III